# EUROPEAN PATENT APPLICATION

(11) **EP 3 522 100 A1**
(43) Date of publication of application: **07.08.2019**
(21) Application number: 17856315.1
(22) Date of filing: 28.09.2017
(51) Int. Cl.: G06Q 50/22

(54) **DEMENTIA PATIENT CARE BURDEN DEGREE DETERMINATION DEVICE, DEMENTIA PATIENT CARE BURDEN DEGREE DETERMINATION METHOD, DEMENTIA PATIENT CARE BURDEN DEGREE DETERMINATION PROGRAM, DEMENTIA TREATMENT EFFECT DETERMINATION DEVICE, DEMENTIA TREATMENT EFFECT DETERMINATION METHOD, AND DEMENTIA TREATMENT EFFECT DETERMINATION PROGRAM**

(30) Priority: 28.09.2016 JP 2016190162
(71) Applicant: Foundation for Biomedical Research and Innovation at Kobe, Kobe-shi Hyogo 650-0047 (JP)
(72) Inventor: KIKUCHI, Takashi, Kobe-shi, Hyogo 650-0047 (JP)
(74) Representative: KATZAROV S.A.
(86) International application number: PCT/JP2017/035149
(87) International publication number: WO 2018/062366

(57) **Abstract**

A dementia care burden level determination device (100) includes: an obtainment unit (110) that obtains answers to respective questions about the behavioral pattern of a patient, the questions each belonging to one of n domains; a score calculator (111) that, in accordance with the answers obtained by the obtainment unit (110), calculates scores for the respective domains, the scores being expressed as numerical values and related to the behavioral pattern of the patient; a distance calculator (112) that calculates a Euclidean distance between fixed-point coordinates and coordinates represented by the scores for the respective domains calculated by the score calculator (111) in an n-dimensional space in which number lines used to show the scores for the respective domains correspond to coordinate axes; a determination unit (113) that determines a dementia care burden level for the patient to be cared for in accordance with the Euclidean distance calculated by the distance calculator (112); and an output unit (114) that outputs the dementia care burden level determined by the determination unit (113).

## Description

### [Technical Field]

The present invention relates to a dementia care burden level determination device for determining a care burden level for a patient with dementia to be cared for and a dementia treatment therapeutic effect determination device for determining a therapeutic effect of treatment for dementia on a patient with dementia.

### [Background Art]

A technique of determining a care burden level for a patient with dementia in accordance with answers to respective questions about activities of daily living of the patient has been known.

For instance, Non-Patent Literature (NPL) 1 discloses a technique by which answers, convertible into numerical values, to respective questions about the behavioral pattern of a patient are obtained, and a care burden level for the patient is determined in accordance with the sum of numerical values into which the answers have been converted.

### [Citation List]

### [Non Patent Literature]

[NPL 1] Validation of the relevant outcome scale for Alzheimer's disease: a novel multidomain assessment for daily medical practice. Alzheimers Res Ther. 2011 Jul 6;3(5):27.

### [Summary of Invention]

### [Technical Problem]

By using the conventional technique, a care burden level (e.g., dementia care burden level) can be determined with certain accuracy. However, the accuracy may not necessarily be sufficient, and thus it is desired that the dementia care burden level be determined more accurately.

The present invention has been made in view of the above problem, and an objective of the present invention is to provide a dementia care burden level determination device, a dementia care burden level determination method, and a dementia care burden level determination program that enable to determine a dementia care burden level more accurately than before and a dementia treatment therapeutic effect determination device, a dementia treatment therapeutic effect determination method, and a dementia treatment therapeutic effect determination program that enable to determine a therapeutic effect of treatment for dementia more accurately than before.

### [Solution to Problem]

To achieve the above objective, a dementia care burden level determination device according to the present invention is a dementia care burden level determination device for determining a dementia care burden level for a patient to be cared for and includes: an obtainment unit that obtains answers to respective questions about the behavioral pattern of the patient, the questions each belonging to one of n domains, where n is an integer of at least two; a score calculator that, in accordance with the answers obtained by the obtainment unit, calculates scores for the respective domains, the scores being expressed as numerical values and related to the behavioral pattern of the patient; a distance calculator that calculates a Euclidean distance between fixed-point coordinates and coordinates represented by the scores for the respective domains calculated by the score calculator in an n-dimensional space in which number lines used to show the scores for the respective domains correspond to coordinate axes; a determination unit that determines a dementia care burden level for the patient to be cared for in accordance with the Euclidean distance calculated by the distance calculator; and an output unit that outputs the dementia care burden level determined by the determination unit.

To achieve the above objective, a dementia care burden level determination method according to the present invention is a dementia care burden level determination method of determining a dementia care burden level for a patient to be cared for and includes: obtaining answers to respective questions about the behavioral pattern of the patient, the questions each belonging to one of n domains, where n is an integer of at least two; in accordance with the answers obtained in the obtaining, calculating scores for the respective domains, the scores being expressed as numerical values and related to the behavioral pattern of the patient; in an n-dimensional space in which number lines used to show the scores for the respective domains correspond to coordinate axes, calculating a Euclidean distance between fixed-point coordinates and coordinates represented by the scores for the respective domains calculated in the calculating; in accordance with the Euclidean distance calculated in the calculating, determining a dementia care burden level for the patient to be cared for; and outputting the dementia care burden level determined in the determining.

To achieve the above objective, a dementia treatment therapeutic effect determination device according to the present invention is a dementia treatment therapeutic effect determination device for determining a therapeutic effect of treatment for dementia in terms of a dementia care burden level for a patient to be cared for and includes: an obtainment unit that obtains answers to respective questions about the behavioral pattern of the patient twice, once the first time round and once the second time round, the questions each belonging to one of n domains, where n is an integer of at least two; a score calculator that, for each round the obtainment unit obtains the answers and in accordance with the answers obtained by the obtainment unit, calculates scores for the respective domains, the scores being expressed as numerical values and related to the behavioral pattern of the patient; a distance calculator that calculates, for each round the score calculator calculates the scores, a Euclidean distance between fixed-point coordinates and coordinates represented by the scores for the respective domains in an n-dimensional space in which number lines used to show the scores for the respective domains correspond to coordinate axes; a determination unit that determines a therapeutic effect of treatment for dementia in terms of the dementia care burden level for the patient to be cared for, in accordance with a difference between the Euclidean distance calculated the first time round by the distance calculator and the Euclidean distance calculated the second time round by the distance calculator; and an output unit that outputs the therapeutic effect of the treatment for dementia determined by the determination unit.

To achieve the above objective, a dementia treatment therapeutic effect determination method according the present invention is a dementia treatment therapeutic effect determination method of determining a therapeutic effect of treatment for dementia in terms of a dementia care burden level for a patient to be cared for and includes: obtaining answers to respective questions about the behavioral pattern of the patient twice, once the first time round and once the second time round, the questions each belonging to one of n domains, where n is an integer of at least two; for each round the answers are obtained in the obtaining and in accordance with the answers obtained in the obtaining, calculating scores for the respective domains, the scores being expressed as numerical values and related to the behavioral pattern of the patient; for each round the scores are calculated in the calculating, calculating a Euclidean distance between fixed-point coordinates and coordinates represented by the scores for the respective domains calculated in the calculating, in an n-dimensional space in which number lines used to show the scores for the respective domains correspond to coordinate axes; in accordance with a difference between the Euclidean distance calculated the first time round in the calculating and the Euclidean distance calculated the second time round in the calculating, determining a therapeutic effect of treatment for dementia in terms of the dementia care burden level for the patient to be cared for; and outputting the therapeutic effect of the treatment for dementia determined in the determining.

### [Advantageous Effects of Invention]

The dementia care burden level determination device and the dementia care burden level determination method according to the present invention enable to determine a dementia care burden level more logically and more accurately than before. In addition, the dementia treatment therapeutic effect determination device and the dementia treatment therapeutic effect determination method according to the present invention enable comprehensive assessment of individual concepts of dementia (e.g., cognitive and memory abilities, activities of daily living, and behavioral and physiological symptoms of a patient with dementia) as a patient's condition and determination of a therapeutic effect based on the comprehensive assessment, which are impossible to be done by the conventional technique.

### [Brief Description of Drawings]

Fig. 1 is a block diagram illustrating the functional configuration of a dementia care burden level determination device.
Fig. 2 is a perspective diagram of a portable terminal.
Fig. 3 illustrates an example of a question image.
Fig. 4 illustrates the data composition of a numerical value reference table.
Fig. 5 illustrates the data composition of a domain reference table.
Fig. 6 illustrates an example of a three-dimensional image.
Fig. 7A is a Euclidean distance histogram for patients diagnosed with a "severe level".
Fig. 7B is a Euclidean distance histogram for patients diagnosed with a "moderate level".
Fig. 7C is a Euclidean distance histogram for patients diagnosed with a "mild level".
Fig. 7D is a Euclidean distance histogram for patients diagnosed with a "stage before requiring dementia care".
Fig. 8A is a cutoff value between the "stage before requiring dementia care" and the "mild level".
Fig. 8B is a cutoff value between the "mild level" and the "moderate level".
Fig. 8C is a cutoff value between the "moderate level" and the "severe level".
Fig. 9A is a cutoff value between the "stage before requiring dementia care" and the "moderate level".
Fig. 9B is a cutoff value between the "stage before requiring dementia care" and the "severe level".
Fig. 9C is a cutoff value between the "mild level" and the "severe level".
Fig. 10 illustrates the probability of accurate determination for each cutoff value.
Fig. 11 is a flowchart of dementia care burden level determination processing.
Fig. 12 illustrates an example of a three-dimensional image.
Fig. 13 illustrates an example of an image displayed on a touch panel.
Fig. 14 is a block diagram illustrating the functional configuration of a dementia treatment therapeutic effect determination device.
Fig. 15 is a flowchart of the dementia treatment therapeutic effect determination processing.
Fig. 16 is a flowchart of Euclidean distance calculation processing.

### [Description of Embodiments]

### (Background regarding how an aspect of the present invention has been conceived)

By using the conventional technique, a dementia care burden level can be determined with certain accuracy. There are primarily three types of factors that affect a care burden level for a patient with dementia, and the three factors are the "cognitive and memory abilities", "activities of daily living", and "behavioral and psychological symptoms" of the patient with dementia. For the conventional technique, however, these factors are individually assessed by conducting respective psychological tests, and the dementia care burden level based on each of the results of the tests is determined. Thus, the result of the determined dementia care burden level differs between the psychological tests conducted. Accordingly, the accuracy is not sufficient. As such, when assessing a patient with dementia by using the three types of factors, the "cognitive and memory abilities", the "activities of daily living", and the "behavioral and psychological symptoms", it is desired that the three factors be assessed comprehensively as a patient's condition, thereby determining a dementia care burden level more accurately. In addition, for the conventional technique, as in the case of the determination of a dementia care burden level, individually assessing the "cognitive and memory abilities", the "activities of daily living", and the "behavioral and psychological symptoms" of a patient with dementia by using different psychological tests is the only way to determine a therapeutic effect of treatment for dementia. Thus, especially in development of a new drug for dementia, a technique that enables comprehensive assessment of these factors has been desired.

Among concepts of dementia, the "cognitive and memory abilities", the "activities of daily living", and the "behavioral and psychological symptoms", which are assessed in the psychological tests, represent different concepts. Thus, scores obtained as a result of conducting the respective psychological tests should be considered as mathematically different vectors. That is, simply adding the scores obtained as a result of conducting the psychological tests and assessing the "cognitive and memory abilities", the "activities of daily living", and the "behavioral and psychological symptoms" is mathematically incorrect as the vectors are considered as scalar quantities, and such values are largely biased. Accordingly, a technique is required by which when assessing, for example, the three factors, the "cognitive and memory abilities", the "activities of daily living", and the "behavioral and psychological symptoms", the factors are treated as three different types of vectors and comprehensively assessed.

With this background, the present invention has been made and provides a technique of comprehensively assessing, as a patient's condition, the concepts of dementia, which have been individually assessed by the conventional technique, such as the "cognitive and memory abilities", the "activities of daily living", and the "behavioral and psychological symptoms". That is, when assessing the three factors, the "cognitive and memory abilities", the "activities of daily living", and the "behavioral and psychological symptoms", scores for the respective factors are represented in three dimensions, and a distance (Euclidean distance) from fixed-point coordinates is measured, which enables to assess the factors as a patient's condition. An objective of the present invention is to provide, by assessing the Euclidean distance and a variation in the Euclidean distance, a dementia care burden level determination device and a dementia care burden level determination method that enable to determine a dementia care burden level more accurately than before and a dementia treatment therapeutic effect determination device and a dementia treatment therapeutic effect determination method that enable to determine a therapeutic effect of treatment for dementia more accurately than before. It should be noted that in this exemplification, the three factors, the "cognitive and memory abilities", the "activities of daily living", and the "behavioral and psychological symptoms", are used. However, the number of factors used in the technique the present invention provides is not limited, and even for the increased number of factors, by using the Euclidean distance, it is possible to determine a dementia care burden level and a therapeutic effect of treatment for dementia in a similar manner.

A dementia care burden level determination device according to an aspect of the present invention is a dementia care burden level determination device for determining a dementia care burden level for a patient to be cared for and includes: an obtainment unit that obtains answers to respective questions about the behavioral pattern of the patient, the questions each belonging to one of n domains, where n is an integer of at least two; a score calculator that, in accordance with the answers obtained by the obtainment unit, calculates scores for the respective domains, the scores being expressed as numerical values and related to the behavioral pattern of the patient; a distance calculator that calculates a Euclidean distance between fixed-point coordinates and coordinates represented by the scores for the respective domains calculated by the score calculator in an n-dimensional space in which number lines used to show the scores for the respective domains correspond to coordinate axes; a determination unit that determines a dementia care burden level for the patient to be cared for in accordance with the Euclidean distance calculated by the distance calculator; and an output unit that outputs the dementia care burden level determined by the determination unit.

Here, the domains each correspond to a group of questions containing a common factor.

For the conventional technique of determining a dementia care burden level, numerical values based on obtained answers are simply treated as scalars. That is, a dementia care burden level is determined in accordance with the sum of the numerical values based on the obtained answers. Thus, even if each question belongs to one of domains, the dementia care burden level does not reflect the information regarding which of the domains each question belongs to. For instance, in the case in which the total score of the previous measurement was 30 points, if one domain scores minus five points from the previous measurement while another domain scores plus five points from the previous measurement, the total score of the current measurement is 30 points, the same total score as that of the previous measurement. However, it is not possible to show the changes in the scores for the respective domains, resulting in a lack of accuracy in determination of a dementia care burden level.

In contrast, for the dementia care burden level determination device according to an aspect of the present invention, a numerical value based on an obtained answer is treated as the vector length of a vector extending in the individual orientation of a domain to which the corresponding question belongs. In the n-dimensional space in which the individual orientations of the domains correspond to the coordinate axes, a dementia care burden level is determined in accordance with the vector length of a difference vector between the sum of vectors for the respective domains and a vector representing the fixed-point coordinates. Accordingly, for the dementia care burden level determination device according to an aspect of the present invention, the dementia care burden level can reflect the information regarding which of the domains each question belongs to and information regarding changes in scores.

Accordingly, the dementia care burden level determination device according to an aspect of the present invention can determine a dementia care burden level more accurately than before.

For instance, the fixed-point coordinates may correspond to coordinates obtained as a result of a combination, for which the dementia care burden level is preset to be highest, of answers to the respective questions, and the determination unit may determine the dementia care burden level so that when two Euclidean distances, one shorter than the other and one longer than the other, are compared, the dementia care burden level for the shorter Euclidean distance is not lower than the dementia care burden level for the longer Euclidean distance.

This makes it possible that the longer the distance from the coordinates for the highest dementia care burden level, the dementia care burden level is determined to be lower.

For instance, the score calculator may calculate the scores by calculating, for each of the domains, the sum of numerical values into which answers to questions belonging to the domain have been converted.

This enables to calculate the scores for the respective domains with a relatively small calculation amount.

For instance, the score calculator may calculate the scores by calculating, for each of the domains, a deviation value expressed as a Z-score or a T-score for the patient in the population distribution of sums calculated for respective patients, the sums each being a sum of numerical values into which answers to questions belonging to the domain have been converted.

Thus, even if the number of questions belonging to each domain differs, it is possible to decrease an effect on a determined result due to the differences in the number of questions belonging to each domain.

For instance, n may be three.

Thus, the space in which the number lines used to show the scores for the respective domains correspond to the coordinate axes is equal to the three-dimensional space of the real world. Accordingly, a user who uses the dementia care burden level determination device can perceive the space in which the number lines used to show the scores for the respective domains correspond to the coordinate axes, in a similar way as the user perceives the space of the real world.

For instance, the dementia care burden level may further include an image generator that generates a three-dimensional image in which the position of the fixed-point coordinates and the position of the coordinates represented by the scores for the respective domains calculated by the score calculator are visible together in the n-dimensional space, in which the output unit may also display the three-dimensional image generated by the image generator.

This enables the user who uses the dementia care burden level determination device to visually identify a positional relationship between the fixed-point coordinates and the coordinates represented by the scores for the respective domains calculated by the score calculator.

For instance, the dementia care burden level determination device may further include: question image storage that stores, for each of the questions, a question image containing the question and multiple answer choices to the question; a reception unit that receives, for each of the question images stored in the question image storage, an answer choice selected from the multiple answer choices contained in the question image; and numerical value storage that stores, for each of the question images stored in the question image storage, numerical values and the multiple answer choices contained in the question image, the numerical values each being associated with a different one of the multiple answer choices, in which the output unit may also display each of the question images stored in the question image storage, the obtainment unit may obtain each answer by obtaining, as an answer, the answer choice received by the reception unit, and the score calculator may calculate the scores in accordance with the numerical values stored in the numerical value storage in association with the answer choices corresponding to the answers obtained by the obtainment unit.

This enables to obtain answers by using the question images.

For instance, each of the question images may contain five answer choices, and each of the question images may also contain three illustrations for three respective answer choices among the five answer choices, the two remaining answer choices being associated with the highest numerical value and the lowest numerical value in the numerical value storage.

This enables the user who uses the dementia care burden level determination device to select an answer choice while referring to the illustrations corresponding to the three answer choices, which are not both extremes.

For instance, the dementia care burden level determination device may further include: a position identifying unit that identifies the position of the dementia care burden level determination device by using a global positioning system (GPS); medical facility storage that stores the positions of medical facilities; and a medical facility identifying unit that, when the dementia care burden level determined by the determination unit is equal to or greater than a predetermined value, identifies a medical facility that the patient is recommended to attend, in accordance with the position of the dementia care burden level determination device identified by the position identifying unit and the positions of the medical facilities stored in the medical facility storage, in which the output unit may also output information regarding the medical facility identified by the medical facility identifying unit.

This enables the user who uses the dementia care burden level determination device to obtain the information regarding the medical facility that the patient is recommended to attend.

For instance, the dementia care burden level determination device may further include: map storage that stores a map; and a route image generator that generates, by using the map stored in the map storage, a route image showing a route to be taken when attending the medical facility identified by the medical facility identifying unit from the position of the dementia care burden level determination device identified by the position identifying unit, in which the output unit may also display the route image generated by the route image generator.

This enables the user who uses the dementia care burden level determination device to visually identify the route to be taken when attending the medical facility that the patient is recommended to attend.

A dementia care burden level determination method according to an aspect of the present invention is a dementia care burden level determination method of determining a dementia care burden level for a patient to be cared for and includes: obtaining answers to respective questions about the behavioral pattern of the patient, the questions each belonging to one of n domains, where n is an integer of at least two; in accordance with the answers obtained in the obtaining, calculating scores for the respective domains, the scores being expressed as numerical values and related to the behavioral pattern of the patient; in an n-dimensional space in which number lines used to show the scores for the respective domains correspond to coordinate axes, calculating a Euclidean distance between fixed-point coordinates and coordinates represented by the scores for the respective domains calculated in the calculating; in accordance with the Euclidean distance calculated in the calculating, determining a dementia care burden level for the patient to be cared for; and outputting the dementia care burden level determined in the determining.

For the conventional technique of determining a dementia care burden level, numerical values based on obtained answers are simply treated as scalars. That is, a dementia care burden level is determined in accordance with the sum of the numerical values based on the obtained answers. Thus, even if each question belongs to one of domains, the dementia care burden level does not reflect the information regarding which of the domains each question belongs to.

In contrast, for the dementia care burden level determination method according to an aspect of the present invention, each numerical value based on an obtained answer is treated as the vector length of a vector extending in the individual orientation of a domain to which the corresponding question belongs. In the n-dimensional space in which the individual orientations of the domains correspond to the coordinate axes, a dementia care burden level is determined in accordance with the vector length of a difference vector between the sum of vectors corresponding to answers and a vector representing the fixed-point coordinates. Accordingly, for the dementia care burden level determination device according to an aspect of the present invention, the dementia care burden level can reflect the information regarding which of the domains each question belongs to.

Accordingly, the dementia care burden level determination method according to an aspect of the present invention can determine a dementia care burden level more accurately than before.

A dementia care burden level determination program according to an aspect of the present invention is a program designed to cause a computer including a processor and memory to implement the dementia care burden level determination method.

For the conventional technique of determining a dementia care burden level, numerical values based on obtained answers are simply treated as scalars. That is, a dementia care burden level is determined in accordance with the sum of the numerical values based on the obtained answers. Thus, even if each question belongs to one of domains, the dementia care burden level does not reflect the information regarding which of the domains each question belongs to and information regarding scores.

In contrast, for a dementia care burden level determination program according to an aspect of the present invention, each numerical value based on an obtained answer is treated as the vector length of a vector extending in the individual orientation of a domain to which the corresponding question belongs. In the n-dimensional space in which the individual orientations of the domains correspond to the coordinate axes, a dementia care burden level is determined in accordance with the vector length of a difference vector between the sum of vectors corresponding to answers and a vector representing the fixed-point coordinates. Accordingly, for the dementia care burden level determination device according to an aspect of the present invention, the dementia care burden level can reflect the information regarding which of the domains each question belongs to and information regarding scores.

Accordingly, the dementia care burden level determination program according to an aspect of the present invention can determine a dementia care burden level more accurately than before.

A dementia treatment therapeutic effect determination device according to an aspect of the present invention is a dementia treatment therapeutic effect determination device for determining a therapeutic effect of treatment for dementia in terms of a dementia care burden level for a patient to be cared for and includes: an obtainment unit that obtains answers to respective questions about the behavioral pattern of the patient twice, once the first time round and once the second time round, the questions each belonging to one of n domains, where n is an integer of at least two; a score calculator that, for each round the obtainment unit obtains the answers and in accordance with the answers obtained by the obtainment unit, calculates scores for the respective domains, the scores being expressed as numerical values and related to the behavioral pattern of the patient; a distance calculator that calculates, for each round the score calculator calculates the scores, a Euclidean distance between fixed-point coordinates and coordinates represented by the scores for the respective domains in an n-dimensional space in which number lines used to show the scores for the respective domains correspond to coordinate axes; a determination unit that determines a therapeutic effect of treatment for dementia in terms of the dementia care burden level for the patient to be cared for, in accordance with a difference between the Euclidean distance calculated the first time round by the distance calculator and the Euclidean distance calculated the second time round by the distance calculator; and an output unit that outputs the therapeutic effect of the treatment for dementia determined by the determination unit.

For the conventional technique of determining a dementia care burden level, numerical values based on obtained answers are simply treated as scalars. That is, a dementia care burden level is determined in accordance with the sum of the numerical values based on the obtained answers. Thus, even if each question belongs to one of domains, a therapeutic effect of treatment for dementia in terms of the dementia care burden level does not reflect the information regarding which of the domains each question belongs to and information regarding scores.

In contrast, for a dementia treatment therapeutic effect determination device according to an aspect of the present invention, each numerical value based on an obtained answer is treated as the vector length of a vector extending in the individual orientation of a domain to which the corresponding question belongs. In the n-dimensional space in which the individual orientations of the domains correspond to the coordinate axes, the vector length of a difference vector between the sum of vectors corresponding to answers and the vector representing the fixed-point coordinates. A therapeutic effect of treatment for dementia in terms of the dementia care burden level is determined in accordance with a difference between the vector length calculated the first time round and the vector length calculated the second time round. Accordingly, for the dementia treatment therapeutic effect determination device according to an aspect of the present invention, the therapeutic effect of the treatment for dementia in terms of the dementia care burden level can reflect the information regarding which of the domains each question belongs to and information regarding scores.

Accordingly, the dementia treatment therapeutic effect determination device according to an aspect of the present invention can determine a therapeutic effect in terms of the dementia care burden level more accurately than before.

For instance, the fixed-point coordinates may correspond to coordinates obtained as a result of a combination, for which the dementia care burden level is preset to be highest, of answers to the respective questions and, the determination unit may determine the therapeutic effect of the treatment for dementia in terms of the dementia care burden level so that when two Euclidean distances, one shorter than the other and one longer than the other, are compared, the therapeutic effect of the treatment for dementia for the shorter Euclidean distance is not lower than the therapeutic effect of the treatment for dementia for the longer Euclidean distance.

This makes it possible that the shorter the Euclidean distance, the therapeutic effect of the treatment for dementia in terms of the dementia care burden level is determined to be smaller.

For instance, the score calculator may calculate the scores by calculating, for each of the domains, the sum of numerical values into which answers to questions belonging to the domain have been converted.

This enables to calculate scores for the respective domains with a relatively small calculation amount.

For instance, the score calculator may calculate the scores by calculating, for each of the domains, a deviation value expressed as a Z-score or a T-score for the patient in the population distribution of sums calculated for respective patients, the sums each being a sum of numerical values into which answers to questions belonging to the domain have been converted.

Thus, even if the number of questions belonging to each domain differs, it is possible to decrease an effect on a determined result due to the differences in the number of questions belonging to each domain.

For instance, n may be three.

Thus, the space in which the number lines used to show the scores for the respective domains correspond to the coordinate axes is equal to the three-dimensional space of the real world. Accordingly, a user who uses the dementia care burden level determination device can perceive the space in which the number lines used to show the scores for the respective domains correspond to the coordinate axes, in a similar way as the user perceives the space of the real world.

For instance, the dementia treatment therapeutic effect determination device may further include an image generator that generates a three-dimensional image in which the position of the fixed-point coordinates, the position of the coordinates represented by the scores for the respective domains calculated the first time round by the score calculator, and the position of the coordinates represented by the scores for the respective domains calculated the second time round by the score calculator are visible together in the n-dimensional space, in which the output unit may also display the three-dimensional image generated by the image generator.

This enables the user who uses the dementia care burden level determination device to visually identify positional relationships between the fixed-point coordinates, the coordinates represented by the scores for the respective domains calculated the first time round by the score calculator, and the coordinates represented by the scores for the respective domains calculated the second time round by the score calculator.

For instance, the dementia treatment therapeutic effect determination device may further include: question image storage that stores, for each of the questions, a question image containing the question and multiple answer choices to the question; a reception unit that receives, for each of the question images stored in the question image storage, an answer choice selected from the multiple answer choices contained in the question image; and numerical value storage that stores, for each of the question images stored in the question image storage, numerical values and the multiple answer choices contained in the question image, the numerical values each being associated with a different one of the multiple answer choices, in which the output unit may also display each of the question images stored in the question image storage, the obtainment unit may obtain each answer by obtaining, as an answer, the answer choice received by the reception unit, and the score calculator may calculate the scores in accordance with the numerical values stored in the numerical value storage in association with the answer choices corresponding to the answers obtained by the obtainment unit.

This enables to obtain answers by using the question images.

For instance, each of the question images may contain five answer choices, and each of the question images may also contain three illustrations for three respective answer choices among the five answer choices, the two remaining answer choices being associated with the highest numerical value and the lowest numerical value in the numerical value storage.

This enables the user who uses the dementia treatment therapeutic effect determination device to select an answer choice while referring to the illustrations corresponding to the three answer choices, which are not both extremes.

A dementia treatment therapeutic effect determination method according to an aspect of the present invention is a dementia treatment therapeutic effect determination method of determining a therapeutic effect of treatment for dementia in terms of a dementia care burden level for a patient to be cared for and includes: obtaining answers to respective questions about the behavioral pattern of the patient twice, once the first time round and once the second time round, the questions each belonging to one of n domains, where n is an integer of at least two; for each round the answers are obtained in the obtaining and in accordance with the answers obtained in the obtaining, calculating scores for the respective domains, the scores being expressed as numerical values and related to the behavioral pattern of the patient; for each round the scores are calculated in the calculating, calculating a Euclidean distance between fixed-point coordinates and coordinates represented by the scores for the respective domains calculated in the calculating, in an n-dimensional space in which number lines used to show the scores for the respective domains correspond to coordinate axes; in accordance with a difference between the Euclidean distance calculated the first time round in the calculating and the Euclidean distance calculated the second time round in the calculating, determining a therapeutic effect of treatment for dementia in terms of the dementia care burden level for the patient to be cared for; and outputting the therapeutic effect of the treatment for dementia determined in the determining.

For the conventional technique of determining a dementia care burden level, numerical values based on obtained answers are simply treated as scalars. That is, a dementia care burden level is determined in accordance with the sum of the numerical values based on the obtained answers. Thus, even if each question belongs to one of domains, a therapeutic effect of treatment for dementia in terms of the dementia care burden level does not reflect the information regarding which of the domains each question belongs to and information regarding scores.

In contrast, for the dementia treatment therapeutic effect determination method according to an aspect of the present invention, each numerical value based on an obtained answer is treated as the vector length of a vector extending in the individual orientation of a domain to which the corresponding question belongs. In the n-dimensional space in which the individual orientations of the domains correspond to the coordinate axes, the vector length of a difference vector between the sum of vectors corresponding to answers and the vector representing the fixed-point coordinates. A therapeutic effect of treatment for dementia is determined in accordance with a difference between the vector length calculated the first time round and the vector length calculated the second time round. Accordingly, for the dementia treatment therapeutic effect determination method according to an aspect of the present invention, the therapeutic effect of the treatment for dementia in terms of the dementia care burden level can reflect the information regarding which of the domains each question belongs to and information regarding scores.

Accordingly, the dementia treatment therapeutic effect determination method according to an aspect of the present invention can determine a therapeutic effect of treatment for dementia in terms of the dementia care burden level more accurately than before.

A dementia treatment therapeutic effect determination program according to an aspect of the present invention is a program designed to cause a computer including a processor and memory to implement the dementia treatment therapeutic effect determination method.

For the conventional technique of determining a therapeutic effect of treatment for dementia, numerical values based on obtained answers are simply treated as scalars. That is, a therapeutic effect of treatment for dementia is determined in accordance with the sum of the numerical values based on the obtained answers. Thus, even if each question belongs to one of domains, the therapeutic effect of the treatment for dementia does not reflect the information regarding which of the domains each question belongs to and information regarding scores.

In contrast, for the dementia treatment therapeutic effect determination program according to an aspect of the present invention, each numerical value based on an obtained answer is treated as the vector length of a vector extending in the individual orientation of a domain to which the corresponding question belongs. In the n-dimensional space in which the individual orientations of the domains correspond to the coordinate axes, the vector length of a difference vector between the sum of vectors corresponding to answers and the vector representing the fixed-point coordinates. A therapeutic effect of treatment for dementia is determined in accordance with a difference between the vector length calculated the first time round and the vector length calculated the second time round. Accordingly, for the dementia treatment therapeutic effect determination program according to an aspect of the present invention, the therapeutic effect of the treatment for dementia can reflect the information regarding which of the domains each question belongs to and information regarding scores.

Accordingly, the dementia treatment therapeutic effect determination program according to an aspect of the present invention can determine a therapeutic effect of treatment for dementia more accurately than before.

### (Embodiment 1)

Hereinafter, a specific example of a dementia care burden level determination device according to an aspect of the present invention is described. It should be noted that all the embodiments described below show preferable specific examples of the present invention. Numerical values, shapes, materials, structural elements, the arrangement and connection of the structural elements, steps, the order of the steps, and others described in the embodiments below are mere examples and are not intended to limit the present invention. The present invention is defined by only Claims. Therefore, among the structural elements in the embodiments below, the structural elements not recited in the independent claims representing the superordinate concepts of the present invention are not necessarily required to address the problems which the present invention faces, but are provided to realize more preferable embodiments.

### <Outline>

A dementia care burden level determination device according to an aspect of the present invention is caused to function by a processor running an application program stored in memory in a portable terminal represented by, for example, a smart phone.

To each of 14 questions about the behavioral pattern of a patient, the dementia care burden level determination device obtains an answer convertible into a numerical value corresponding to one of 1 point, 3 points, 5 points, 7 points, and 9 points, the 14 questions each belonging to one of three domains.

The dementia care burden level determination device calculates, as a score for each domain, the sum of numerical values into which the answers have been converted.

In a three-dimensional space in which number lines used to show the scores for the respective domains correspond to coordinate axes, the dementia care burden level determination device then calculates a Euclidean distance between coordinates represented by the scores for the respective domains and fixed-point coordinates (here, coordinates in the case in which every answer to every question scores the lowest point).

The dementia care burden level determination device determines a dementia care burden level for the patient in accordance with the calculated Euclidean distance.

Hereinafter, the dementia care burden level determination device is described in detail with reference to the Drawings.

### <Configuration>

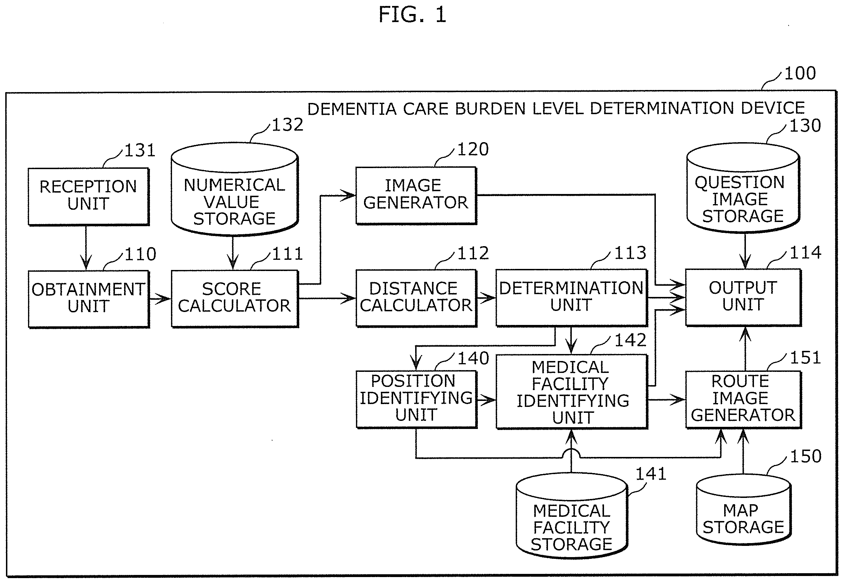

Fig. 1 is a block diagram illustrating the functional configuration of dementia care burden level determination device 100 according to Embodiment 1.

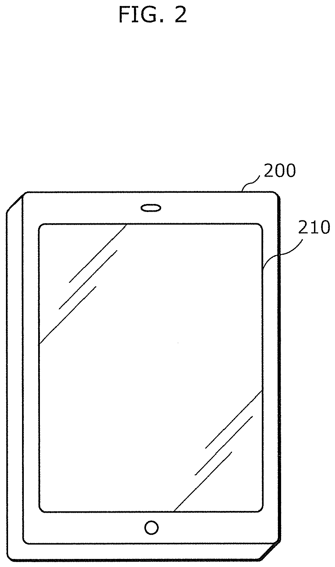

Dementia care burden level determination device 100 determines a dementia care burden level for a patient to be cared for. For example, dementia care burden level determination device 100 is caused to function by a (not shown) processor running an application program stored in (not shown) memory in portable terminal 200 illustrated in Fig. 2. Here, the processor and the memory are parts of portable terminal 200.

Moreover, for example, input to dementia care burden level determination device 100 is performed through a touch operation on touch panel 210, and output from dementia care burden level determination device 100 is performed by displaying an image on touch panel 210. Here, touch panel 210 is a part of portable terminal 200.

As illustrated in Fig. 1, dementia care burden level determination device 100 includes obtainment unit 110, score calculator 111, distance calculator 112, determination unit 113, output unit 114, image generator 120, question image storage 130, reception unit 131, numerical value storage 132, position identifying unit 140, medical facility storage 141, medical facility identifying unit 142, map storage 150, and route image generator 151.

For each of multiple (here, 14) questions about the behavioral pattern of the patient, question image storage 130 stores a question image containing the question and multiple (here, five) answer choices to the question.

As an example, question image storage 130 serves as part of a memory storage region in portable terminal 200.

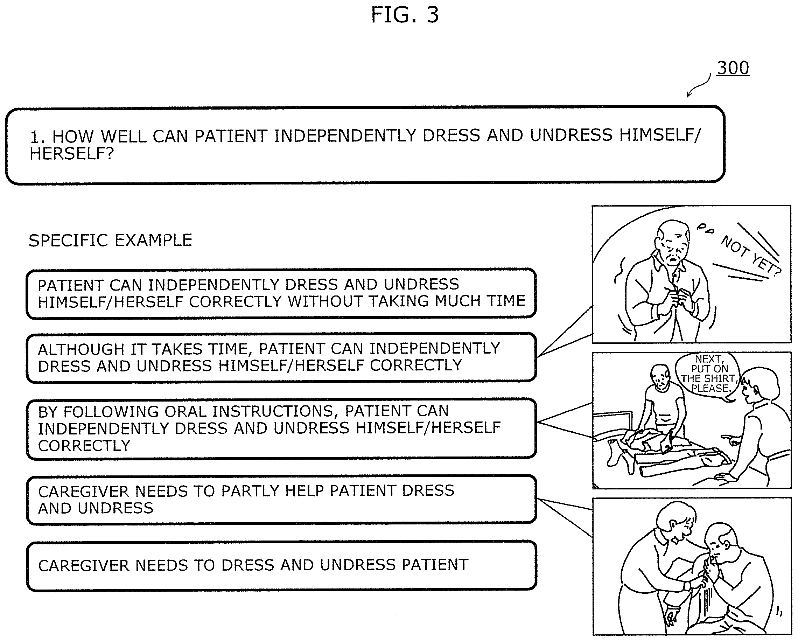

Fig. 3 illustrates question image 300 as an example of a question image stored in question image storage 130.

As illustrated in Fig. 3, question image 300 contains a question represented by text "HOW WELL CAN PATIENT INDEPENDENTLY DRESS AND UNDRESS HIMSELF/HERSELF?" and five answer choices represented by text, "PATIENT CAN INDEPENDENTLY DRESS AND UNDRESS HIMSELF/HERSELF CORRECTLY WITHOUT TAKING MUCH TIME", "ALTHOUGH IT TAKES TIME, PATIENT CAN INDEPENDENTLY DRESS AND UNDRESS HIMSELF/HERSELF CORRECTLY", "BY FOLLOWING ORAL INSTRUCTIONS, PATIENT CAN INDEPENDENTLY DRESS AND UNDRESS HIMSELF/HERSELF CORRECTLY", "CAREGIVER NEEDS TO PARTLY HELP PATIENT DRESS AND UNDRESS", and "CAREGIVER NEEDS TO DRESS AND UNDRESS PATIENT".

Each of the five hierarchical answer choices is associated with a numerical value in accordance with numerical value reference table 400 stored in numerical value storage 132, which is described later. Here, "PATIENT CAN INDEPENDENTLY DRESS AND UNDRESS HIMSELF/HERSELF CORRECTLY WITHOUT TAKING MUCH TIME" is associated with "9". "ALTHOUGH IT TAKES TIME, PATIENT CAN INDEPENDENTLY DRESS AND UNDRESS HIMSELF/HERSELF CORRECTLY" is associated with "7". "BY FOLLOWING ORAL INSTRUCTIONS, PATIENT CAN INDEPENDENTLY DRESS AND UNDRESS HIMSELF/HERSELF CORRECTLY" is associated with "5". "CAREGIVER NEEDS TO PARTLY HELP PATIENT DRESS AND UNDRESS" is associated with "3". "CAREGIVER NEEDS TO DRESS AND UNDRESS PATIENT" is associated with "1".

In addition, among the five answer choices, question image 300 contains illustrations for the three respective answer choices, "ALTHOUGH IT TAKES TIME, PATIENT CAN INDEPENDENTLY DRESS AND UNDRESS HIMSELF/HERSELF CORRECTLY" associated with the second highest numerical value "7", "BY FOLLOWING ORAL INSTRUCTIONS, PATIENT CAN INDEPENDENTLY DRESS AND UNDRESS HIMSELF/HERSELF CORRECTLY" associated with the third highest numerical value "5", and "CAREGIVER NEEDS TO PARTLY HELP PATIENT DRESS AND UNDRESS" associated with the fourth highest numerical value "3".

Referring to Fig. 1 again, the description of dementia care burden level determination device 100 continues.

For each of the question images stored in question image storage 130, numerical value storage 132 stores numerical values and the five answer choices contained in the question image, the numerical values each being associated with a different one of the five answer choices.

As an example, numerical value storage 132 serves as part of a memory storage region in portable terminal 200.

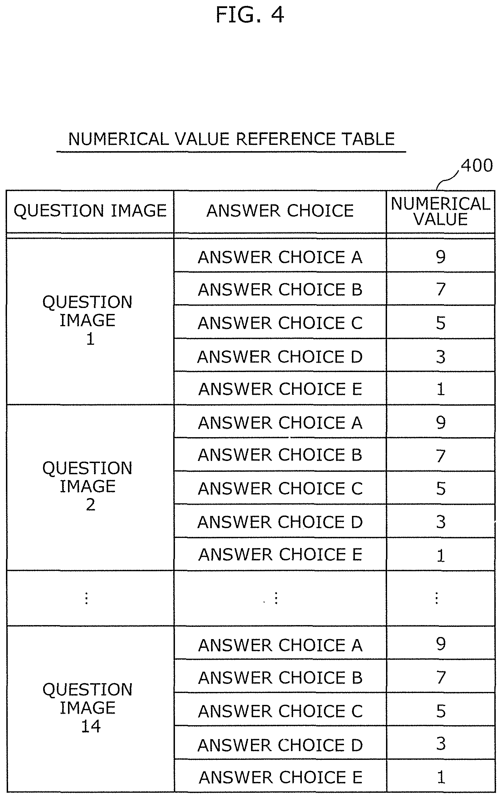

Fig. 4 illustrates the data composition of numerical value reference table 400 as an example of a numerical value reference table stored in numerical value storage 132.

As illustrated in Fig. 4, for each of the question images stored in question image storage 130, numerical value reference table 400 associates each of the five answer choices contained in the question image with a numerical value (specifically, one of "9", "7", "5", "3", and "1"). Here, the answer choices are associated with the respective numerical values so that answer choices for which a burden imposed on a caregiver who cares for the patient tends to increase are associated with smaller numerical values.

For each of the question images stored in question image storage 130, numerical value storage 132 stores a question contained in the question image and a domain to which the question belongs, in association with each other.

Here, the domain corresponds to a group of questions containing a common factor.

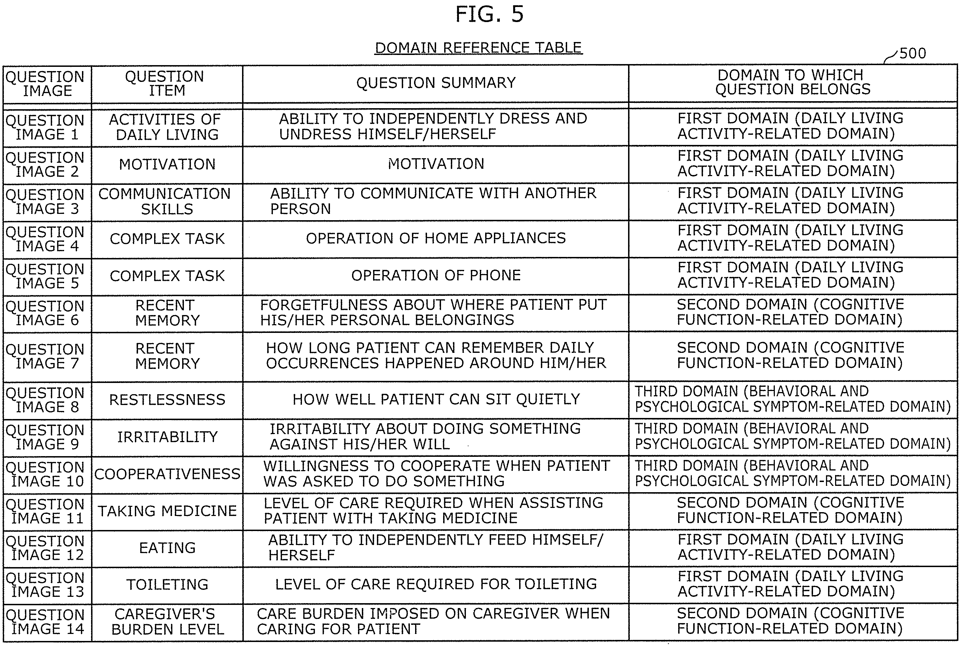

Fig. 5 illustrates the data composition of domain reference table 500 as an example of a domain reference table stored in numerical value storage 132.

As illustrated in Fig. 5, in domain reference table 500, for each of the question images stored in question image storage 130, a question contained in the question image, the question item of the question, the question summary of the question, and a domain to which the question belongs are associated with each other.

Here, the questions contained in question images 1, 2, 3, 4, 5, 12, and 13 belong to a first domain (daily living activity-related domain), the questions contained in question images 6, 7, 11, and 14 belong to a second domain (cognitive function-related domain), and the questions contained in question images 8, 9, and 10 belong to a third domain (behavioral and psychological symptom-related domain).

Referring to Fig. 1 again, the description of dementia care burden level determination device 100 continues.

For each of the question images stored in question image storage 130, reception unit 131 receives an answer choice selected from the five answer choices contained in the question image.

As an example, reception unit 131 is caused to function by running a program stored in the memory on the processor and controlling touch panel 210, in portable terminal 200.

In a state in which a question image is displayed on touch panel 210 by output unit 114, which is described later, by a user performing a touch operation on an answer-choice display region, reception unit 131 receives the answer choice as a selected answer choice.

Obtainment unit 110 obtains answers to the 14 respective questions about the behavioral pattern of the patient, the 14 questions each belonging to one of n (here, three) domains. That is, obtainment unit 110 obtains 14 answer choices received by reception unit 131, as respective answers.

As an example, obtainment unit 110 is caused to function by the processor running a program stored in the memory in portable terminal 200.

In accordance with the answers obtained by obtainment unit 110, score calculator 111 calculates scores for the respective domains, the scores being expressed as numerical values and related to the behavioral pattern of the patient. That is, score calculator 111 converts the answers obtained by obtainment unit 110 into numerical values by referring to numerical value reference table 400 and domain reference table 500, which are stored in numerical value storage 132. Score calculator 111 then calculates, as a score for each domain, the sum of the numerical values into which the answers have been converted.

As an example, score calculator 111 is caused to function by the processor running a program stored in the memory in portable terminal 200.

Image generator 120 generates a three-dimensional image in which the position of the fixed-point coordinates and the position of coordinates represented by the scores for the respective domains calculated by score calculator 111 are visible together in the three-dimensional space in which the number lines used to show the scores for the respective domains correspond to the coordinate axes.

Here, the fixed-point coordinates correspond to coordinates represented when an answer choice associated with the smallest numerical value (here, "1") is selected for every question. Specifically, the coordinates in which (first domain, second domain, third domain) is equal to (7, 4, 3) correspond to the fixed-point coordinates.

As an example, image generator 120 is caused to function by the processor running a program stored in the memory in portable terminal 200.

In the three-dimensional space in which the number lines used to show the scores for the respective domains correspond to the coordinate axes, distance calculator 112 calculates a Euclidean distance between the fixed-point coordinates and the coordinates represented by the scores for the respective domains calculated by score calculator 111. The Euclidean distance is equal to the vector length of a difference vector between a three-dimensional vector representing the fixed-point coordinates and a three-dimensional vector representing the coordinates represented by the scores for the respective domains calculated by score calculator 111.

As an example, distance calculator 112 is caused to function by the processor running a program stored in the memory in portable terminal 200.

Determination unit 113 determines a dementia care burden level for the patient in accordance with the Euclidean distance calculated by distance calculator 112. That is, determination unit 113 stores three thresholds and compares the three thresholds and the Euclidean distance calculated by distance calculator 112. (1) When the Euclidean distance is less than a first threshold, determination unit 113 determines the dementia care burden level to be "severe". (2) When the Euclidean distance is equal to or greater than the first threshold and less than a second threshold, determination unit 113 determines the dementia care burden level to be "moderate". (3) When the Euclidean distance is equal to or greater than the second threshold and less than a third threshold, determination unit 113 determines the dementia care burden level to be "mild". (4) When the Euclidean distance is equal to or greater than the third threshold, determination unit 113 determines the dementia care burden level to be a "stage before requiring dementia care".

As an example, determination unit 113 is caused to function by the processor running a program stored in the memory in portable terminal 200.

Hereinafter, a specific way to determine the three thresholds stored in determination unit 113 is described.

First, 427 patients diagnosed by doctors with one of the dementia care burden levels, the "severe level", the "moderate level", the "mild level", and the "stage before requiring dementia care" were asked 14 questions, by using the 14 question images stored in question image storage 130. Here, the 14 questions are contained in the respective question images.

Obtainment unit 110 obtained answers from the 427 patients, score calculator 111 calculated scores for the respective 427 patients, and distance calculator 112 calculated Euclidean distances for the respective 427 patients.

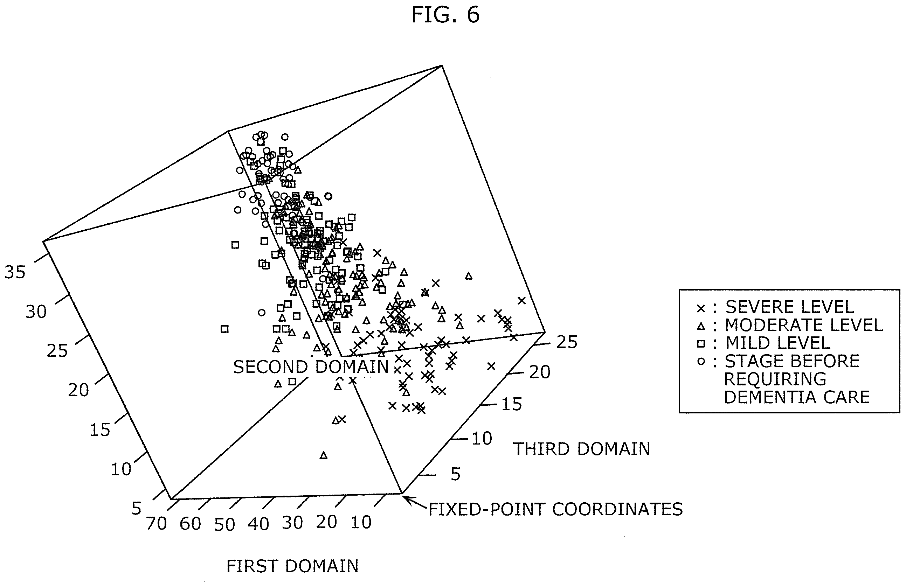

Fig. 6 illustrates an example of a three-dimensional image generated by image generator 120, and in the three-dimensional image, the position of the fixed-point coordinates and the positions of coordinates represented by the scores for the respective 427 patients calculated by score calculator 111 are visible together.

In Fig. 6, the coordinates represented by "x" correspond to a group of patients diagnosed by the doctors with the "severe" dementia care burden level. The coordinates represented by "Δ" correspond to a group of patients diagnosed by the doctors with the "moderate" dementia care burden level. The coordinates represented by "□" correspond to a group of patients diagnosed by the doctors with the "mild" dementia care burden level. The coordinates represented by "○" correspond to a group of patients diagnosed by the doctors with the "stage before requiring dementia care" as their dementia care burden level.

Fig. 6 shows that, regarding each of the coordinates calculated by the score calculator, there is a correlation between the Euclidean distance from the fixed-point coordinates and the dementia care burden level with which the patient was diagnosed by the doctor.

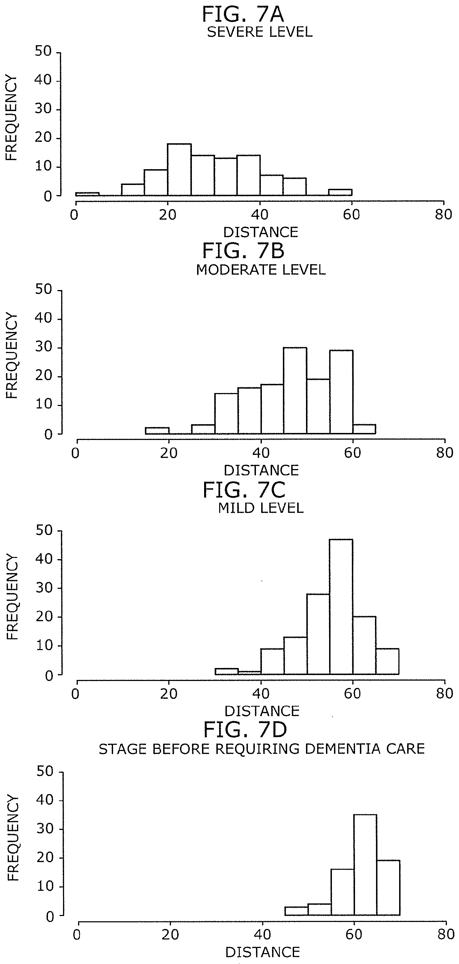

Fig. 7A illustrates a histogram of the Euclidean distances calculated by distance calculator 112, for the group of the patients diagnosed by the doctors with the (a) "severe" dementia care burden level. Fig. 7B illustrates a histogram of the Euclidean distances calculated by distance calculator 112, for the group of the patients diagnosed by the doctors with the (b) "moderate" dementia care burden level. Fig. 7C illustrates a histogram of the Euclidean distances calculated by distance calculator 112, for the group of the patients diagnosed by the doctors with the (c) "mild" dementia care burden level. Fig. 7D is a histogram of the Euclidean distances calculated by distance calculator 112, for the group of the patients diagnosed by the doctors with the (d) "stage before requiring dementia care" as their dementia care burden level.

Figs. 7A to 7D also show that, regarding each of the coordinates calculated by the score calculator, there is a correlation between the Euclidean distance from the fixed-point coordinates and the dementia care burden level with which the patient was diagnosed by the doctor.

As a result of conducting analysis of variance on these Euclidean distances, it was found that the average values of the Euclidean distances for the respective patient groups show a significant difference of P < 0.0001.

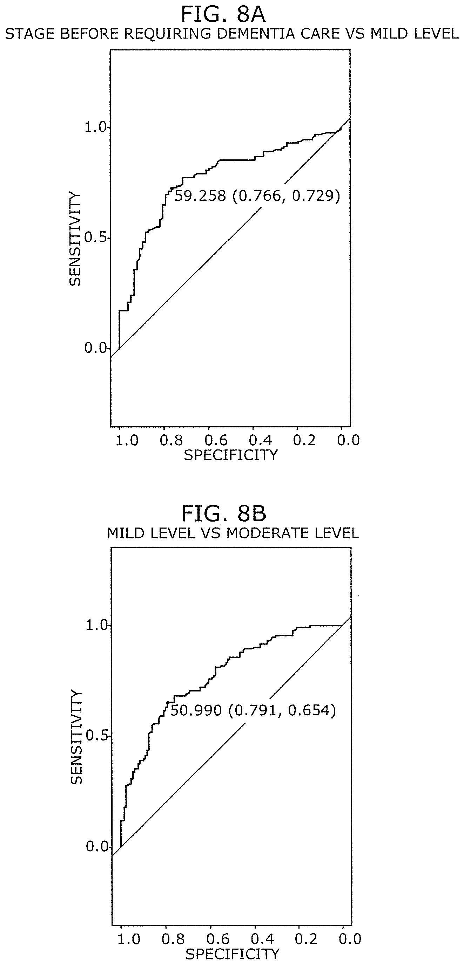

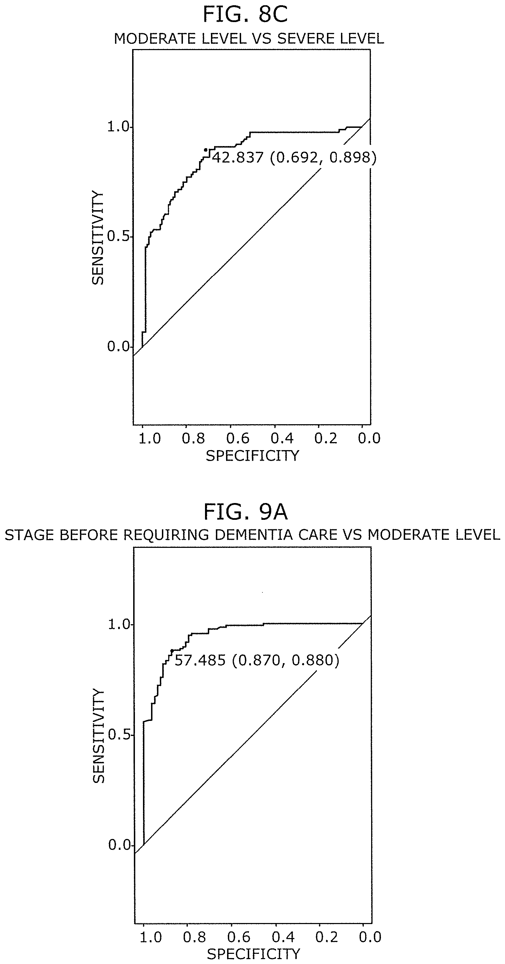

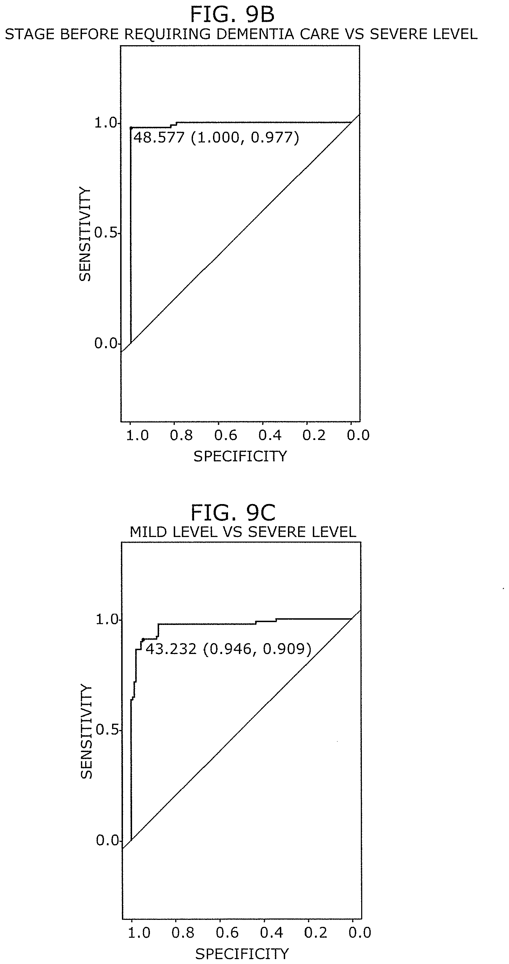

Next, by a receiver operating characteristic (ROC) method, a cutoff value between the "stage before requiring dementia care" and the "mild level", a cutoff value between the "mild level" and the "moderate level", a cutoff value between the "moderate level" and the "severe level", a cutoff value between the "stage before requiring dementia care" and the "moderate level", a cutoff value between the "mild level" and the "severe level", and a cutoff value between the "stage before requiring dementia care" and the "severe level" were estimated by using the Euclidean distance. Figs. 8A to 8C and Figs. 9A to 9C illustrate the estimated cutoff values.

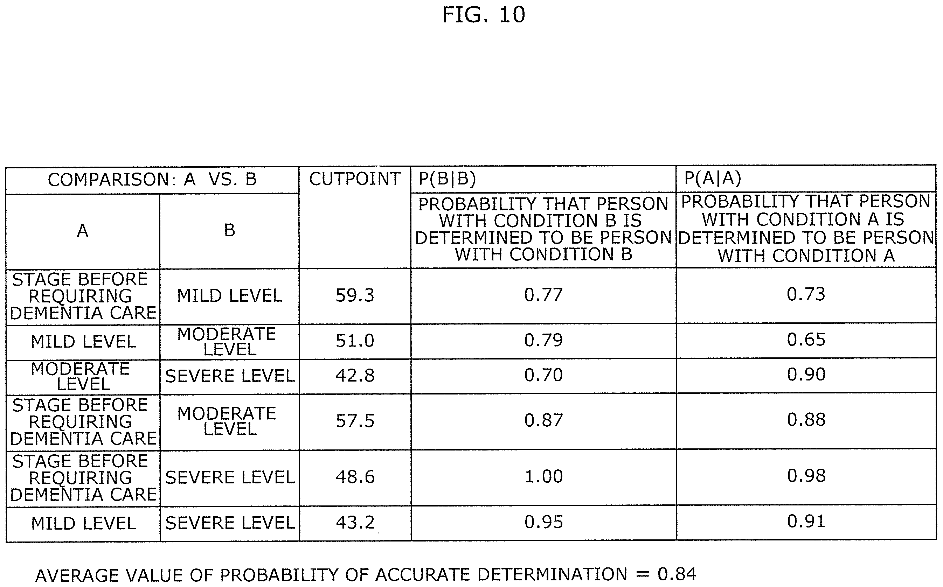

In addition, the probability of accurate determination for each of the estimated cutoff values was calculated. Here, the probability of accurate determination means the probability that a patient of an X dementia care burden level is determined to be the patient of the X dementia care burden level. Fig. 10 illustrates a table showing the calculated results. As a result of the calculation, the average value of the probability of accurate determination in every combination is 0.84. When limiting to the cutoff values between the close groups of patients (i.e., between the "stage before requiring dementia care" and the "mild level", between the "mild level" and the "moderate level", and between the "moderate level" and the "severe level"), the average value of the probability of accurate determination is 0.76, which means a good determination accuracy level.

In accordance with these results, the first threshold is set to be "42.8", the second threshold is set to be "51.0", and the third threshold is set to be "59.3".

Referring to Fig. 1 again, the description of dementia care burden level determination device 100 continues.

Position identifying unit 140 identifies the position of dementia care burden level determination device 100 by using a global positioning system (GPS).

As an example, position identifying unit 140 is caused to function by running a program stored in the memory on the processor and controlling a position identifying device that is an internal device of portable terminal 200 and that uses the GPS, in portable terminal 200.

Medical facility storage 141 stores the positions of medical facilities. That is, medical facility storage 141 stores the positons of medical facilities where a person who will require dementia care can be examined. Here, in addition to the position of each medical facility, medical facility storage 141 stores pieces of information such as the name and contact address of the medical facility and the closest station (or bus stop) to the medical facility.

As an example, medical facility storage 141 serves as part of a memory storage region in portable terminal 200.

If the dementia care burden level determined by determination unit 113 is equal to or greater than a predetermined value (e.g., the "mild level", the "moderate level", or the "severe level"), medical facility identifying unit 142 identifies a medical facility that the patient is recommended to attend, in accordance with the position of dementia care burden level determination device 100 identified by position identifying unit 140 and the positions of medical facilities stored in medical facility storage 141. That is, as the medical facility that the patient is recommended to attend, medical facility identifying unit 142 identifies the medical facility closest to dementia care burden level determination device 100, from the medical facilities stored in medical facility storage 141.

As an example, medical facility identifying unit 142 is caused to function by the processor running a program stored in the memory in portable terminal 200.

Map storage 150 stores maps.

As an example, map storage 150 is caused to function by the processor running a program stored in the memory in portable terminal 200.

By using a map stored in map storage 150, route image generator 151 generates a route image showing a route to be taken when attending the medical facility identified by medical facility identifying unit 142 from the position of dementia care burden level determination device 100 identified by position identifying unit 140.

As an example, route image generator 151 is caused to function by the processor running a program stored in the memory in portable terminal 200.

Output unit 114 outputs the dementia care burden level determined by determination unit 113. That is, when determination unit 113 has determined the dementia care burden level to be one of the "severe level", the "moderate level", the "mild level", and the "stage before requiring dementia care", output unit 114 causes touch panel 210 to display an image showing the determined result (e.g., an image containing text showing the determined result).

As an example, output unit 114 is caused to function by running a program stored in the memory on the processor and controlling touch panel 210, in portable terminal 200.

In addition, output unit 114 displays the three-dimensional image generated by image generator 120. That is, when image generator 120 has generated a three-dimensional image, output unit 114 causes touch panel 210 to display the three-dimensional image.

Output unit 114 displays each of the question images stored in question image storage 130. That is, in accordance with a touch operation performed on touch panel 210 by the user, output unit 114 causes touch panel 210 to display each of the question images stored in question image storage 130.

In addition, output unit 114 outputs pieces of information regarding the medical facility identified by medical facility identifying unit 142. That is, when medical facility identifying unit 142 has identified a medical facility, output unit 114 causes touch panel 210 to display the name and contact address of the medical facility and the closest station to the medical facility.

Output unit 114 then displays the route image generated by route image generator 151. That is, when route image generator 151 has generated a route image, output unit 114 causes touch panel 210 to display the route image.

Hereinafter, characteristic operation of dementia care burden level determination device 100 having such a configuration is described with reference to the Drawings.

### < Operation>

As its characteristic operation, dementia care burden level determination device 100 performs dementia care burden level determination processing.

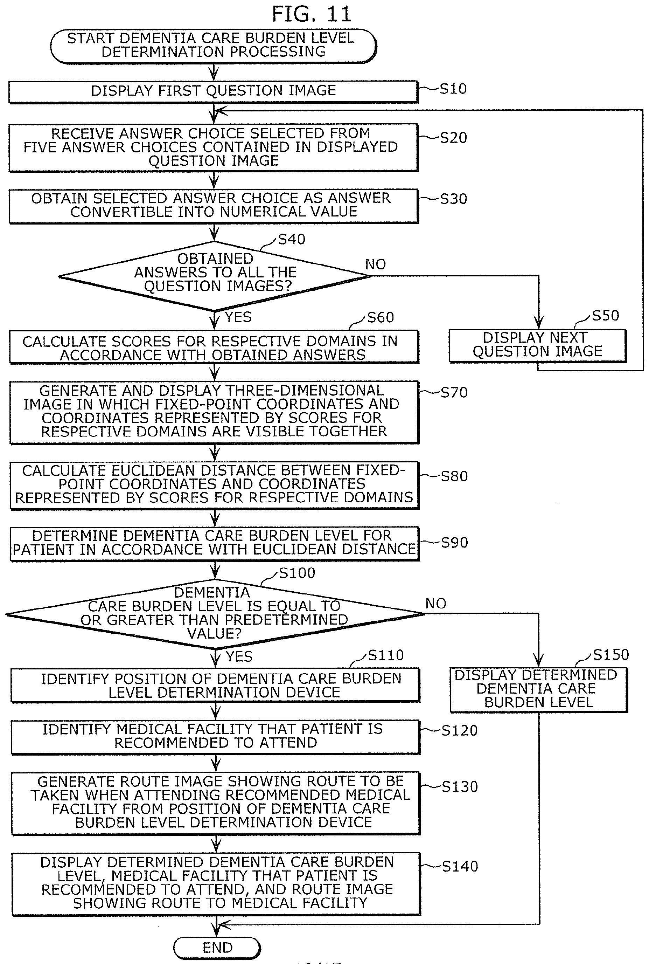

Fig. 11 is a flowchart of the dementia care burden level determination processing.

The dementia care burden level determination processing starts when the user who uses dementia care burden level determination device 100 has performed a touch operation to start the dementia care burden level determination processing on touch panel 210.

When the dementia care burden level determination processing starts, output unit 114 causes touch panel 210 to display the first question image among the 14 question images stored in question image storage 130 (step S10).

When the question image is displayed, reception unit 131 receives an answer choice selected from the five answer choices through a touch operation by the user, the five answer choices being contained in the question image being displayed (step S20).

When reception unit 131 has received the selected answer choice, obtainment unit 110 obtains the selected answer choice as an answer (step S30).

When obtaining the answer, obtainment unit 110 checks whether answers to all the question images stored in question image storage 130 have been obtained (step S40).

In the processing of step S40, if not all the answers to all the question images are obtained (step S40: No), output unit 114 causes touch panel 210 to display the next question image among the 14 question images stored in question image storage 130 (step S50). The dementia care burden level determination processing starts from the processing of step S20 again.

In the processing of step S40, if answers to all the question images are obtained (step S40: Yes), score calculator 111 calculates scores for the respective domains in accordance with the obtained answers, by referring to numerical value reference table 400 and domain reference table 500, which are stored in numerical value storage 132 (step S60).

When the scores for the respective domains are calculated, image generator 120 generates a three-dimensional image in which the fixed-point coordinates and coordinates represented by the scores for the respective domains are visible together in the three-dimensional space in which the number lines used to show the scores for the respective domains correspond to the coordinate axes. Output unit 114 causes touch panel 210 to display the generated three-dimensional image (step S70).

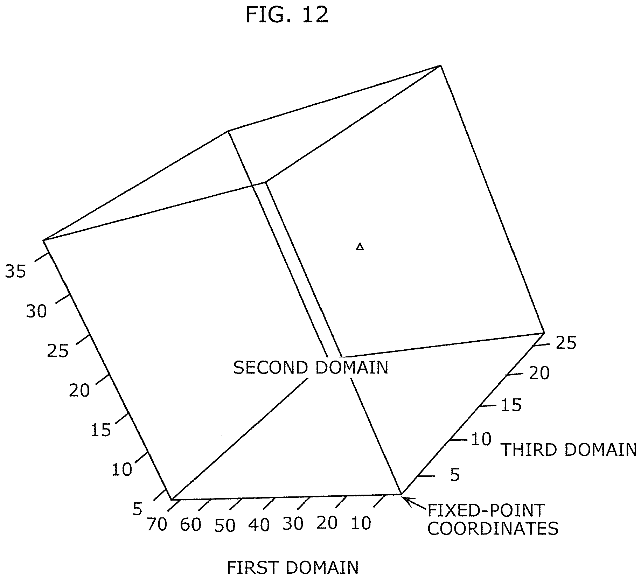

Fig. 12 illustrates an example of the three-dimensional image displayed on touch panel 210 in the processing of step S70.

Referring to Fig. 11 again, the description of the dementia care burden level determination processing continues.

When the three-dimensional image is displayed, distance calculator 112 calculates a Euclidean distance between the fixed-point coordinates and the coordinates represented by the scores for the respective domains (step S80).

When the Euclidean distance is calculated, determination unit 113 determines a dementia care burden level for the patient in accordance with the Euclidean distance (step S90). That is, (1) when the Euclidean distance is less than a first threshold of 43.8, determination unit 113 determines the dementia care burden level to be "severe". (2) When the Euclidean distance is equal to or greater than a first threshold of 43.8 and less than a second threshold of 51.0, determination unit 113 determines the dementia care burden level to be "moderate". (3) When the Euclidean distance is equal to or greater than a second threshold of 51.0 and less than a third threshold of 59.3, determination unit 113 determines the dementia care burden level to be "mild". (4) When the Euclidean distance is equal to or greater than a third threshold of 59.3, determination unit 113 determines the dementia care burden level to be the "stage before requiring dementia care".

Furthermore, determination unit 113 checks whether the determined dementia care burden level is equal to or greater than a predetermined value, that is, whether the determined result is the "mild level", the "moderate level", or the "severe level" (step S100).

In the processing of step S100, if the determined dementia care burden level is equal to or greater than the predetermined value (step S100: Yes), position identifying unit 140 identifies the position of dementia care burden level determination device 100 by using the GPS (step S110).

When the position of dementia care burden level determination device 100 is identified, in accordance with the position of dementia care burden level determination device 100 and the positions of the medical facilities stored in medical facility storage 141, medical facility identifying unit 142 identifies the closest medical facility to the position of dementia care burden level determination device 100 as a medical facility that the patient is recommended to attend (step S120).

When the medical facility is identified, by using a map stored in map storage 150, route image generator 151 generates a route image showing a route to be taken when attending the identified medical facility from the position of dementia care burden level determination device 100 (step S130).

When the route image is generated, output unit 114 causes touch panel 210 to display the dementia care burden level determined by determination unit 113, the medical facility identified by medical facility identifying unit 142, and the route image that has been generated by route image generator 151 and that shows the route to the medical facility (step S140).

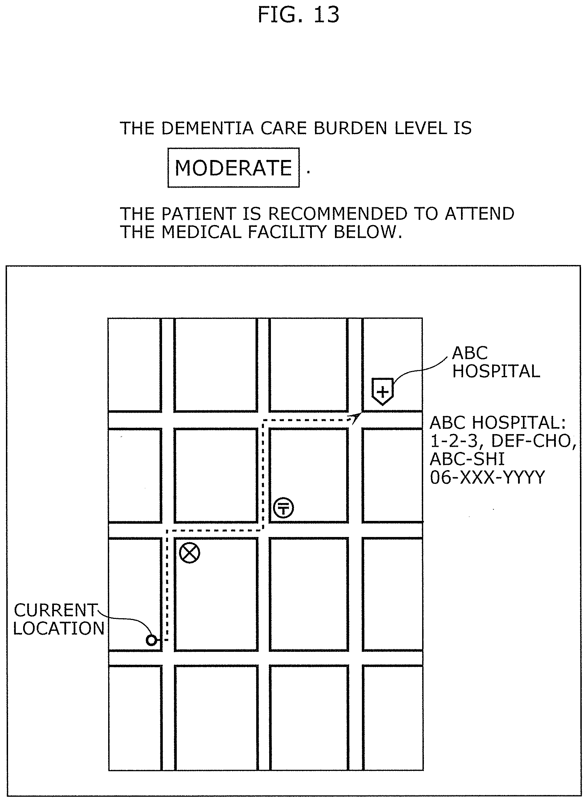

Fig. 13 illustrates an example of an image displayed on touch panel 210 in the processing of step S140. In this instance, the image illustrated in Fig. 13 is an example of an image displayed when the determined dementia care burden level is "moderate" and the identified medical facility is "ABC hospital".

As illustrated in Fig. 13, the image displayed on touch panel 210 contains pieces of information such as the determined dementia care burden level (here, "moderate level"), the name (here, "ABC hospital") and contact address of the identified medical facility, and the route to be taken when attending the identified medical facility from the position of dementia care burden level determination device 100.

Referring to Fig. 11 again, the description of the dementia care burden level determination processing continues.

In the processing of step S100, if the determined dementia care burden level is not equal to or greater than the predetermined value (step S100: No), output unit 114 causes touch panel 210 to display the dementia care burden level determined by determination unit 113 (step S150).

When the processing of step S140 ends and when the processing of step S150 ends, dementia care burden level determination device 100 ends the dementia care burden level determination processing.

### < Consideration>

For the conventional technique of determining a dementia care burden level, numerical values based on obtained answers are simply treated as scalars. Thus, even if each question belongs to one of domains, the dementia care burden level does not reflect the information regarding which of the domains each question belongs to.

For instance, in case (1), the sum of answers to respective questions belonging to a first domain is 10, the sum of answers to respective questions belonging to a second domain is 10, and the sum of answers to respective questions belonging to a third domain is 10. In case (2), the sum of answers to the respective questions belonging to the first domain is five, the sum of answers to the respective questions belonging to the second domain is five, and the sum of answers to the respective questions belonging to the third domain is 20. In both cases, for the conventional technique of determining a dementia care burden level, the sum of the answers to all the questions is 30. Thus, when determining the dementia care burden level, case (1) and case (2) cannot be treated separately.

In contrast, for dementia care burden level determination device 100 described above, a numerical value based on an obtained answer is treated as the vector length of a vector extending in the individual orientation of a domain to which the corresponding question belongs. Thus, the dementia care burden level can reflect the information regarding which of the domains each question belongs to.

For instance, in case (1), the sum of answers to the respective questions belonging to the first domain is 10, the sum of answers to the respective questions belonging to the second domain is 10, and the sum of answers to the respective questions belonging to the third domain is 10. In case (2), the sum of answers to the respective questions belonging to the first domain is five, the sum of answers to the respective questions belonging to the second domain is five, and the sum of answers to the respective questions belonging to the third domain is 20. For dementia care burden level determination device 100, different coordinates are obtained for case (1) and case (2). Accordingly, when determining the dementia care burden level, case (1) and case (2) can be treated separately. This enables to determine differences in the dementia care burden level between the domains, which enables to optimize, for example, the locations of facilities that provide nursing care services. Furthermore, for instance, creating dementia care burden level database enables to show the number of patients with dementia, the level of nursing care required by each of the patients with dementia, and where the patients with dementia reside. This enables to provide adequate nursing care services in accordance with information regarding how many and where care workers are needed.

### (Embodiment 2)

Hereinafter, a specific example of a dementia treatment therapeutic effect determination device according to an aspect of the present invention is described. It should be noted that all the embodiments below show preferable specific examples of the present invention. Numerical values, shapes, materials, structural elements, the arrangement and connection of the structural elements, steps, the order of the steps, and others described in the embodiments below are mere examples and are not intended to limit the present invention. The present invention is defined by only Claims. Therefore, among the structural elements in the embodiments below, the structural elements not recited in the independent claims representing the superordinate concepts of the present invention are not necessarily required to address the problems which the present invention faces, but are provided to realize more preferable embodiments.

### <Outline>

As in the case of dementia care burden level determination device 100 in Embodiment 1, a dementia treatment therapeutic effect determination device according to an aspect of the present invention is caused to function by a processor running an application program stored in memory in a portable terminal represented by, for example, a smart phone.

The hardware configuration of the dementia treatment therapeutic effect determination device is similar to that of dementia care burden level determination device 100 in Embodiment 1. However, the software of the dementia treatment therapeutic effect determination device is partially changed from that of dementia care burden level determination device 100 in Embodiment 1. Thus, the dementia treatment therapeutic effect determination device has functions partially changed from those of dementia care burden level determination device 100 in Embodiment 1.

Dementia care burden level determination device 100 in Embodiment 1 obtains answers and calculates scores for the respective domains in accordance with the obtained answers. In the three-dimensional space in which the number lines used to show the scores for the respective domains correspond to the coordinate axes, dementia care burden level determination device 100 calculates a Euclidean distance between the fixed-point coordinates and coordinates represented by the scores for the respective domains and determines the dementia care burden level for a patient in accordance with the calculated Euclidean distance.

In contrast, the dementia treatment therapeutic effect determination device obtains answers twice with an interval between the first obtainment and the second obtainment and, for each round the dementia treatment therapeutic effect determination device obtains the answers, calculates scores for respective domains in accordance with the obtained answers. The dementia treatment therapeutic effect determination device then determines a therapeutic effect of treatment for dementia in terms of a dementia care burden level for a patient in accordance with a difference between a Euclidean distance based on the answers obtained the first time round and a Euclidean distance based on the answers obtained the second time round.

As an example in which the dementia treatment therapeutic effect determination device is used, the following example is considered. The dementia treatment therapeutic effect determination device, for example, obtains a first round of answers before a patient receives medical treatment, such as administration of medicine, and a second round of answers after the patient receives the medical treatment. The dementia treatment therapeutic effect determination device then determines a therapeutic effect of the treatment for dementia in terms of the dementia care burden level for the patient. By using the dementia treatment therapeutic effect determination device in this way, a user who uses the dementia treatment therapeutic effect determination device can know, for example, how effective the medical treatment, such as administration of medicine, have been to reduce the burden of dementia care for the patient.

Hereinafter, the dementia treatment therapeutic effect determination device is described in detail with reference to the Drawings.

### <Configuration>

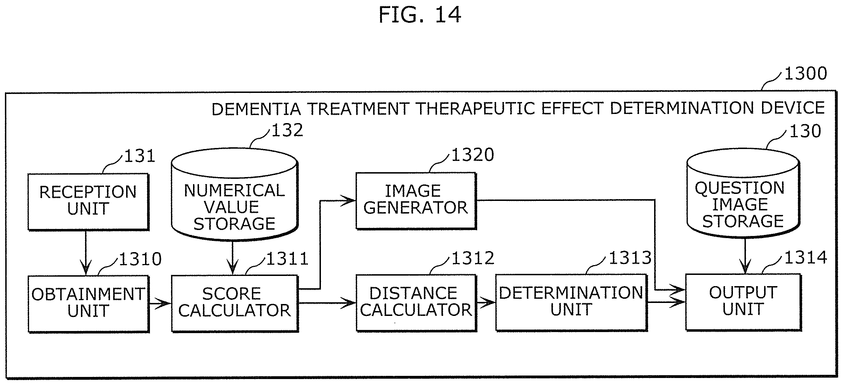

Fig. 14 is a block diagram illustrating the functional configuration of dementia treatment therapeutic effect determination device 1300 according to Embodiment 2.

Dementia treatment therapeutic effect determination device 1300 determines a therapeutic effect of treatment for dementia in terms of a dementia care burden level for a patient to be cared for. As in the case of dementia care burden level determination device 100 in Embodiment 1, for instance, dementia treatment therapeutic effect determination device 1300 is caused to function by a (not shown) processor running an application program stored in (not shown) memory in portable terminal 200 illustrated in Fig. 2. Here, the processor and the memory are parts of portable terminal 200.

In addition, as in the case of dementia care burden level determination device 100 in Embodiment 1, for instance, input to dementia care burden level determination device 100 is performed through a touch operation on touch panel 210, and output from dementia care burden level determination device 100 is performed by displaying output information on touch panel 210. Here, touch panel 210 is a part of portable terminal 200.

As illustrated in Fig. 14, the configuration of dementia treatment therapeutic effect determination device 1300 is created by removing position identifying unit 140, medical facility storage 141, medical facility identifying unit 142, map storage 150, and route image generator 151 from dementia care burden level determination device 100 in Embodiment 1 (see Fig. 1), replacing obtainment unit 110 with obtainment unit 1310, replacing score calculator 111 with score calculator 1311, replacing distance calculator 112 with distance calculator 1312, replacing determination unit 113 with determination unit 1313, replacing output unit 114 with output unit 1314, and replacing image generator 120 with image generator 1320.

Here, the configuration of dementia treatment therapeutic effect determination device 1300 is described, focusing on differences from dementia care burden level determination device 100 in Embodiment 1.

Obtainment unit 1310 obtains twice answers to 14 respective questions about the behavioral pattern of the patient, the 14 questions each belonging to one of n (here, three) domains.

As an example, obtainment unit 1310 is caused to function by the processor running a program stored in the memory in portable terminal 200.

For each round obtainment unit 1311 obtains the answers, in accordance with the answers obtained by obtainment unit 1310, score calculator 1311 calculates scores for the respective domains, the scores being expressed as numerical values and related to the behavioral pattern of the patient. That is, when obtainment unit 1310 has obtained the first round of answers, score calculator 1311 calculates the first round of scores based on the first round of answers, and when obtainment unit 1310 has obtained the second round of answers, score calculator 1311 calculates the second round of scores based on the second round of answers.

As an example, score calculator 1311 is caused to function by the processor running a program stored in the memory in portable terminal 200.

Image generator 1320 generates a three-dimensional image in which the position of the fixed-point coordinates, the position of coordinates represented by the scores for the respective domains calculated the first time round by score calculator 1311, and the position of coordinates represented by the scores for the respective domains calculated the second time round by score calculator 1311 are visible together in a three-dimensional space in which number lines used to show the scores for the respective domains correspond to coordinate axes.

As an example, image generator 1320 is caused to function by the processor running a program stored in the memory in portable terminal 200.

For each round score calculator 1311 calculates the scores, distance calculator 1312 calculates a Euclidean distance between the fixed-point coordinates and the coordinates represented by the scores for the respective domains calculated by score calculator 1311 in the three-dimensional space in which the number lines used to show the scores for the respective domains correspond to the coordinate axes. That is, when obtainment unit 1310 has obtained the first round of answers, distance calculator 1312 calculates the first Euclidean distance based on the first round of answers, and when obtainment unit 1310 has obtained the second round of answers, distance calculator 1312 calculates the second Euclidean distance based on the second round of answers.

As an example, distance calculator 1312 is caused to function by the processor running a program stored in the memory in portable terminal 200.

In accordance with a difference between the Euclidean distance calculated the first time round by distance calculator 1312 and the Euclidean distance calculated the second time round by distance calculator 1312, determination unit 1313 determines a therapeutic effect of treatment for dementia in terms of the dementia care burden level for the patient. Specifically, as a therapeutic effect of the treatment for dementia in terms of the dementia care burden level for the patient, determination unit 1313 calculates the value of the difference between the Euclidean distance obtained the first time round and the Euclidean distance obtained the second time round.

As an example, determination unit 1313 is caused to function by the processor running a program stored in the memory in portable terminal 200.

Output unit 1314 outputs the therapeutic effect of the treatment for dementia determined by determination unit 1313. That is, when determination unit 1313 has determined a therapeutic effect of the treatment for dementia, output unit 1314 causes touch panel 210 to display an image showing the determined result.

As an example, output unit 1314 is caused to function by running a program stored in the memory on the processor and controlling touch panel 210, in portable terminal 200.

In addition, output unit 1314 displays the three-dimensional image generated by the image generator. That is, when image generator 1320 has generated a three-dimensional image, output unit 1314 causes touch panel 210 to display the three-dimensional image.

Output unit 1314 displays each of question images stored in question image storage 130. That is, output unit 1314 causes touch panel 210 to display each of the question images stored in question image storage 130 in accordance with a touch operation performed by a user on touch panel 210.

Hereinafter, characteristic operation of dementia treatment therapeutic effect determination device 1300 having such a configuration is described with reference to the Drawings.

### <Operation>

As its characteristic operation, dementia treatment therapeutic effect determination device 1300 performs dementia treatment therapeutic effect determination processing.

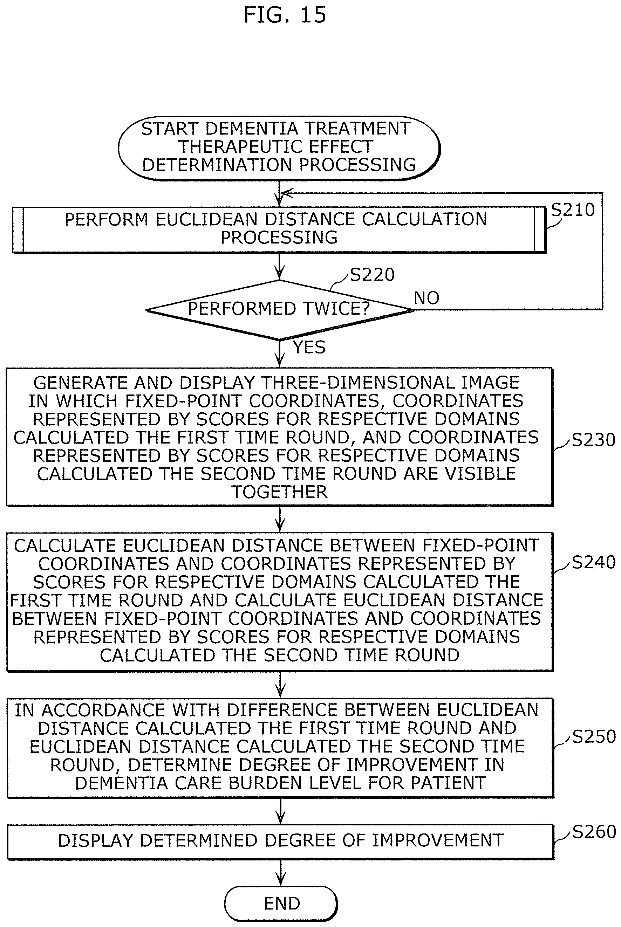

Fig. 15 is a flowchart of the dementia treatment therapeutic effect determination processing.

The dementia treatment therapeutic effect determination processing starts when the user who uses dementia treatment therapeutic effect determination device 1300 has performed a touch operation to start the dementia treatment therapeutic effect determination processing on touch panel 210.

When the dementia treatment therapeutic effect determination processing starts, dementia treatment therapeutic effect determination device 1300 performs Euclidean distance calculation processing (step S210).

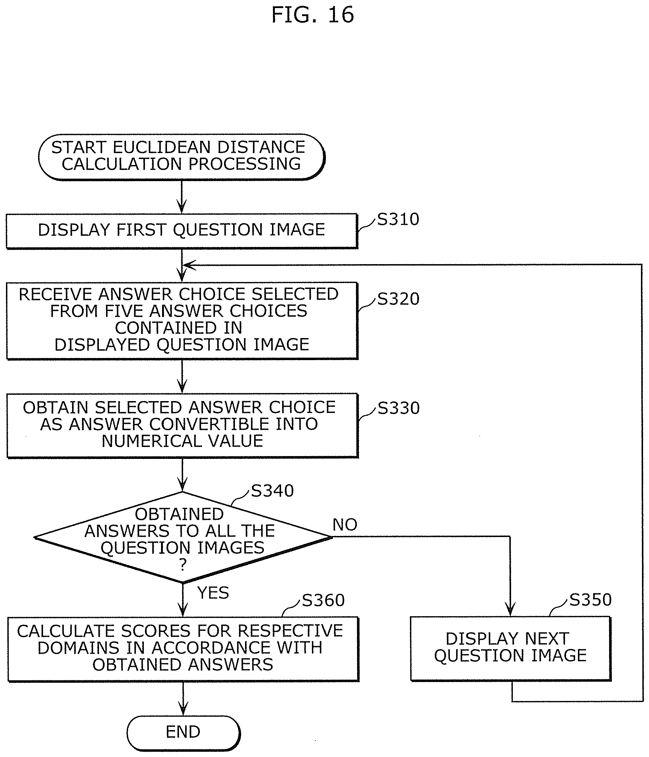

Fig. 16 is a flowchart of the Euclidean distance calculation processing.

The Euclidean distance calculation processing is similar to a series of steps S10 to S60 in the dementia care burden level determination processing in Embodiment 1 (see Fig. 11).

Thus, the description below focuses on differences from the series of steps S10 to S60 in the dementia care burden level determination processing.

The processing of step S310 is performed by output unit 1314 instead of output unit 114 that performs the processing of step S10 in the dementia care burden level determination processing.

The processing of step S320 is similar to that of step S20 in the dementia care burden level determination processing.

The processing of step S330 is performed by obtainment unit 1310 instead of obtainment unit 110 that performs the processing of step S30 in the dementia care burden level determination processing.

The processing of step S340 is performed by obtainment unit 1310 instead of obtainment unit 110 that performs the processing of step S40 in the dementia care burden level determination processing.

The processing of step S350 is performed by output unit 1314 instead of output unit 114 that performs the processing of step S50 in the dementia care burden level determination processing.

The processing of step S360 is performed by score calculator 1311 instead of score calculator 111 that performs the processing of step S60 in the dementia care burden level determination processing.

When the processing of step S360 ends, dementia treatment therapeutic effect determination device 1300 ends the dementia treatment therapeutic effect determination processing.

Referring to Fig. 15 again, the description of the dementia treatment therapeutic effect determination processing continues.

When the Euclidean distance calculation processing is performed in step S210, score calculator 1311 checks whether the Euclidean distance calculation processing has been performed twice (step S220).

In the processing of step S220, if the Euclidean distance calculation processing has not been performed twice (step S220: No), the dementia treatment therapeutic effect determination processing starts from the processing of S210 again.

In the processing of step S220, if the Euclidean distance calculation processing has been performed twice (step S220: Yes), image generator 1320 generates a three-dimensional image in which the fixed-point coordinates, coordinates represented by the scores for the respective domains calculated the first time round by score calculator 1311, coordinates represented by the scores for the respective domains calculated the second time round by score calculator 1311 are visible together in the three-dimensional space in which the number lines used to show the scores for the respective domains correspond to the coordinate axes. Output unit 1314 causes touch panel 210 to display the generated three-dimensional image (step S230).

When the three-dimensional image is displayed, distance calculator 1312 calculates a Euclidean distance between the fixed-point coordinates and the coordinates represented by the scores for the respective domains calculated the first time round and a Euclidean distance between the fixed-point coordinates and the coordinates represented by the scores for the respective domains calculated the second time round, in the three-dimensional space in which the number lines used to show the scores for the respective domains correspond to the coordinate axes (step S240).

When the two Euclidean distances are calculated, in accordance with a difference between the Euclidean distance calculated the first time round by distance calculator 1312 and the Euclidean distance calculated the second time round by distance calculator 1312, determination unit 1313 determines a therapeutic effect of treatment for dementia in terms of the dementia care burden level for a patient (step S250).

When the therapeutic effect of the treatment for dementia is determined, output unit 1314 causes touch panel 210 to display the therapeutic effect of the treatment for dementia determined by determination unit 1313 (step S260).

When the processing of step S260 ends, dementia treatment therapeutic effect determination device 1300 ends the dementia treatment therapeutic effect determination processing.

### (Supplementary explanation)

In Embodiment 1, dementia care burden level determination device 100 according to Embodiment 1 is described. In Embodiment 2, dementia treatment therapeutic effect determination device 1300 according to Embodiment 2 is described.

However, dementia care burden level determination device 100 and dementia treatment therapeutic effect determination device 1300 can be, for example, changed as blow and are not limited to exactly the same as those described in the embodiments, as a matter of course.
(1) As an example, for dementia care burden level determination device 100 in Embodiment 1, in accordance with answers obtained by obtainment unit 110, score calculator 111 calculates, as a score for each domain, the sum of numerical values into which answers corresponding to the domain have been converted. However, as long as, in accordance with answers obtained by obtainment unit 110, score calculator 111 can calculate scores expressed as numerical values for the respective domains, the dementia care burden level determination device 100 does not necessarily have to be exactly the same as that described above. For instance, in accordance with answers obtained by obtainment unit 110, score calculator 111 may calculate, as a score for each domain, a deviation value expressed as a Z-score or a T-score for a patient in the population distribution of sums calculated for respective patients, the sums each being a sum of numerical values into which answers corresponding to the domain have been converted. Examples, including this one, may be considered.
   By taking such a configuration, even if the number of questions belonging to each domain differs, it is possible to decrease an effect on a determined result due to the differences in the number of questions belonging to each domain.
   As another example, in accordance with answers obtained by obtainment unit 110, score calculator 111 may calculate, as a score for each domain, a numerical value by weighting the sum of numerical values into which answers corresponding to the domain have been converted.
   Taking such a configuration enables to control the individual magnitudes of effects of the domains on the determined result.
   Likewise, as an example, for dementia treatment therapeutic effect determination device 1300 in Embodiment 2, in accordance with answers obtained by obtainment unit 1310, score calculator 1311 calculates, as a score for each domain, the sum of numerical values into which answers corresponding to the domain have been converted. However, as long as, in accordance with answers obtained by obtainment unit 1310, score calculator 1311 can calculate scores expressed as numerical values for the respective domains, dementia treatment therapeutic effect determination device 1300 does not necessarily have to be exactly the same as that described above. For instance, in accordance with answers obtained by obtainment unit 1310, score calculator 1311 may calculate, as a score for each domain, a deviation value expressed as a Z-score or a T-score for a patient in the population distribution of sums calculated for respective patients, the sums each being a sum of numerical values into which answers corresponding to the domain have been converted. Examples, including this one, may be considered.
   By taking such a configuration, even if the number of questions belonging to each domain differs, it is possible to decrease an effect on a determined result due to the differences in the number of questions belonging to each domain.
   As another example, in accordance with answers obtained by obtainment unit 1310, score calculator 1311 may calculate, as a score for each domain, a numerical value by weighting the sum of numerical values into which answers corresponding to the domain have been converted.
   Taking such a configuration enables to control the individual magnitudes of effects of the domains on the determined result.
(2) In Embodiment 1, as an example, dementia care burden level determination device 100 is caused to function by a processor running an application program stored in memory in portable terminal 200. However, as long as a function similar to that of dementia care burden level determination device 100 illustrated in Fig. 2 is provided, dementia care burden level determination device 100 does not necessarily have to be caused to function by a processor running an application program stored in memory in portable terminal 200. As an example, dementia care burden level determination device 100 may be caused to function by a processor running an application program stored in memory in a personal computer. As another example, a part of or all of the structural elements illustrated in Fig. 2 may be caused to function by dedicated hardware. Examples, including these, may be considered.
   Likewise, in Embodiment 2, as an example, dementia treatment therapeutic effect determination device 1300 is caused to function by a processor running an application program stored in memory in portable terminal 200. However, as long as a function similar to that of dementia treatment therapeutic effect determination device 1300 illustrated in Fig. 14 is provided, dementia treatment therapeutic effect determination device 1300 does not necessarily have to be caused to function by a processor running an application program stored in memory in portable terminal 200. As an example, dementia treatment therapeutic effect determination device 1300 may be caused to function by a processor running a program stored in memory in a personal computer. As another example, a part of or all of the structural elements illustrated in Fig. 14 may be caused to function by dedicated hardware. Examples, including these, may be considered.
(3) As an example, for dementia care burden level determination device 100 in Embodiment 1 and dementia treatment therapeutic effect determination device 1300 in Embodiment 2, the number of domains to which the questions contained in respective question image 300 belong is three. However, as long as the number of domains to which the questions contained in the question images belong is two or more, the number of domains does not necessarily have to be three. For instance, the number of domains to which the questions contained in respective question image 300 belong may be four. Examples, including this one, may be considered.
(4) As an example, for dementia care burden level determination device 100 in Embodiment 1 and dementia treatment therapeutic effect determination device 1300 in Embodiment 2, respective question image 300 contains five answer choices. However, as long as respective question image 300 contains two or more answer choices, the number of answer choices does not necessarily have to be five. For instance, respective question image 300 may contain three answer choices. Examples, including this one, may be considered.
(5) As an example, for dementia care burden level determination device 100 in Embodiment 1 and dementia treatment therapeutic effect determination device 1300 in Embodiment 2, answer choices are associated with respective numerical values of 1, 3, 5, 7, and 9. However, as long as the answer choices are associated with respective numerical values, the answer choices do not necessarily have to be associated with respective numerical values of 1, 3, 5, 7, and 9. For instance, answer choices may be associated with respective numerical values of 0, 1, 2, 4, and 8. Examples, including this one, may be considered.
(6) As an example, for dementia care burden level determination device 100 in Embodiment 1 and dementia treatment therapeutic effect determination device 1300 in Embodiment 2, obtainment unit 110 and obtainment unit 1310 obtain answer choices received by reception unit 131 as answers. However, as long as answers can be obtained, obtainment unit 110 and obtainment unit 1310 do not necessarily have to obtain answer choices received by reception unit 131 as answers. For instance, obtainment unit 110 and obtainment unit 1310 may each have an input/output interface with which and from which nonvolatile memory is attachable and detachable and obtain answers from the nonvolatile memory attached to the input/output interface thereof. Examples, including this one, may be considered.
(7) As an example, for dementia care burden level determination device 100 in Embodiment 1, medical facility identifying unit 142 identifies the medical facility closest to the position of dementia care burden level determination device 100 as a medical facility that a patient is recommended to attend. However, as long as a medical facility that a patient is recommended to attend can be identified in accordance with a predetermined algorithm, the medical facility closest to the position of dementia care burden level determination device 100 does not necessarily have to be identified as a medical facility that the patient is recommended to attend. For instance, multiple medical facilities located within a certain distance from the position of dementia care burden level determination device 100 may be identified. Examples, including this one, may be considered.
(8) In Embodiment 1, route image 1100 illustrated in Fig. 11 shows the shortest route to be taken when attending the medical facility from the position of dementia care burden level determination device 100. However, as long as route image 1100 shows a recommendable route as a suitable route to be taken when attending the medical facility from the position of dementia care burden level determination device 100, route image 1100 does not necessarily have to show the shortest route from the position of dementia care burden level determination device 100 to the medical facility as a route to be taken when attending the medical facility. For instance, route image 1100 may show a route that is not the shortest route but is a route that enables, by taking public transportation, such as a train or a bus, the patient to arrive at the medical facility faster than when walking to the destination along the shortest route. Examples, including this one, may be considered.
(9) As an example, for dementia treatment therapeutic effect determination device 1300 in Embodiment 2, determination unit 1313 calculates the value of a difference between a Euclidean distance calculated the first time round and a Euclidean distance calculated the second time round, itself, as a therapeutic effect of treatment for dementia in terms of a dementia care burden level for a patient. However, as long as a calculated therapeutic effect of treatment for dementia is based on the value of a difference between a Euclidean distance calculated the first time round and a Euclidean distance calculated the second time round, determination unit 1313 does not necessarily have to calculate the value of the difference between the Euclidean distance calculated the first time round and the Euclidean distance calculated the second time round, itself, as a therapeutic effect of the treatment for dementia. Examples, including the following example, may be considered. Determination unit 1313 may pre-store three positive thresholds. If the value of the difference is less than a first threshold, determination unit 1313 may determine the therapeutic effect of the treatment for dementia to be a "pre-improvement condition". If the value of the difference is equal to or greater than the first threshold and less than a second threshold, determination unit 1313 may determine the therapeutic effect of the treatment for dementia to be "small". If the value of the difference is equal to or greater than the second threshold and less than a third threshold, determination unit 1313 may determine the therapeutic effect of the treatment for dementia to be "intermediate". If the value of the difference is equal to or greater than the third threshold, determination unit 1313 may determine the therapeutic effect of the treatment for dementia to be "great".
(10) In Embodiment 1, with reference to Fig. 6, the coordinate axes used to show scores for the respective domains appear to be coordinate axes in an orthogonal coordinate system. However, the coordinate axes used to show scores for the respective domains do not necessarily have to be limited to orthogonal coordinate axes, but may be oblique coordinate axes.
(11) The embodiments may be realized by the methods described above. The embodiments may also be realized by a computer program designed to cause a computer to implement these methods or by digital signals of the computer program. The embodiments may be realized by recording the computer program or digital signals on a computer-readable recording medium, such as a flexible disk, a hard disk, CD-ROM, an MO disk, a DVD, DVD-ROM, DVD-RAM, a BD, or semiconductor memory. The embodiments may be realized by the digital signals recorded on the recording medium. The embodiments may be realized by transmitting the computer program or digital signals via, for example, a wired or wireless communication line, a network represented by the internet, or data broadcasting. A single computer or two or more computers may run the program. That is, a single computer may perform centralized processing, or multiple computers may perform distributed processing.

### [Industrial Applicability]

The present invention is widely applicable to systems that perform processing related to determination of a dementia care burden level.

### [Reference Signs List]

- 100: dementia care burden level determination device
- 110: obtainment unit
- 111: score calculator
- 112: distance calculator
- 113: determination unit
- 114: output unit
- 120: image generator
- 130: question image storage
- 131: reception unit
- 132: numerical value storage
- 140: position identifying unit
- 141: medical facility storage
- 142: medical facility identifying unit
- 150: map storage
- 151: route image generator
- 1300: dementia treatment therapeutic effect determination device
- 1310: obtainment unit
- 1311: score calculator
- 1312: distance calculator
- 1313: determination unit
- 1314: output unit
- 1320: image generator

## Claims

1. A dementia care burden level determination device for determining a dementia care burden level for a patient to be cared for, the dementia care burden level determination device comprising:
an obtainment unit configured to obtain answers to respective questions about a behavioral pattern of the patient, the questions each belonging to one of n domains, where n is an integer of at least two;
a score calculator that, in accordance with the answers obtained by the obtainment unit, calculates scores for the respective domains, the scores being expressed as numerical values and related to the behavioral pattern of the patient;
a distance calculator that calculates a Euclidean distance between fixed-point coordinates and coordinates represented by the scores for the respective domains calculated by the score calculator in an n-dimensional space in which number lines used to show the scores for the respective domains correspond to coordinate axes;
a determination unit configured to determine a dementia care burden level for the patient to be cared for in accordance with the Euclidean distance calculated by the distance calculator; and
an output unit configured to output the dementia care burden level determined by the determination unit.

2. The dementia care burden level determination device according to claim 1,
wherein the fixed-point coordinates correspond to coordinates obtained as a result of a combination, for which the dementia care burden level is preset to be highest, of answers to the respective questions, and
the determination unit determines the dementia care burden level so that when two of the Euclidean distances, one shorter than the other and one longer than the other, are compared, the dementia care burden level for the shorter Euclidean distance is not lower than the dementia care burden level for the longer Euclidean distance.

3. The dementia care burden level determination device according to claim 1 or 2,
wherein the score calculator calculates the scores by calculating, for each of the domains, a sum of numerical values into which answers to questions belonging to the domain have been converted.

4. The dementia care burden level determination device according to claim 1 or 2,
wherein the score calculator calculates the scores by calculating, for each of the domains, a deviation value expressed as a Z-score or a T-score for the patient in a population distribution of sums calculated for respective patients, the sums each being a sum of numerical values into which answers to questions belonging to the domain have been converted.

5. The dementia care burden level determination device according to any one of claims 1 to 4,
wherein n is three.

6. The dementia care burden level determination device according to any one of claims 1 to 5, further comprising
an image generator that generates a three-dimensional image in which a position of the fixed-point coordinates and a position of the coordinates represented by the scores for the respective domains calculated by the score calculator are visible together in the n-dimensional space,
wherein the output unit also displays the three-dimensional image generated by the image generator.

7. The dementia care burden level determination device according to any one of claims 1 to 6, further comprising:
question image storage that stores, for each of the questions, a question image containing the question and a plurality of answer choices to the question;
a reception unit configured to receive, for each of the question images stored in the question image storage, an answer choice selected from the plurality of answer choices contained in the question image; and
numerical value storage that stores, for each of the question images stored in the question image storage, numerical values and the plurality of answer choices contained in the question image, the numerical values each being associated with a different one of the plurality of answer choices,
wherein the output unit also displays each of the question images stored in the question image storage,
the obtainment unit obtains each answer by obtaining, as an answer, the answer choice received by the reception unit, and
the score calculator calculates the scores in accordance with the numerical values stored in the numerical value storage in association with the answer choices corresponding to the answers obtained by the obtainment unit.

8. The dementia care burden level determination device according to claim 7,
wherein each of the question images contains five answer choices, and
each of the question images also contains three illustrations for three respective answer choices among the five answer choices, the two remaining answer choices being associated with the highest numerical value and the lowest numerical value in the numerical value storage.

9. The dementia care burden level determination device according to any one of claims 1 to 8, further comprising:
a position identifying unit configured to identify a position of the dementia care burden level determination device by using a global positioning system (GPS);
medical facility storage that stores positions of medical facilities; and
a medical facility identifying unit configured, when the dementia care burden level determined by the determination unit is equal to or greater than a predetermined value, to identify a medical facility that the patient is recommended to attend, in accordance with the position of the dementia care burden level determination device identified by the position identifying unit and the positions of the medical facilities stored in the medical facility storage,
wherein the output unit also outputs information regarding the medical facility identified by the medical facility identifying unit.

10. The dementia care burden level determination device according to claim 9, further comprising:
map storage that stores a map; and
a route image generator that generates, by using the map stored in the map storage, a route image showing a route to be taken when attending the medical facility identified by the medical facility identifying unit from the position of the dementia care burden level determination device identified by the position identifying unit,
wherein the output unit also displays the route image generated by the route image generator.

11. A dementia care burden level determination method of determining a dementia care burden level for a patient to be cared for, the dementia care burden level determination method comprising:
obtaining answers to respective questions about a behavioral pattern of the patient, the questions each belonging to one of n domains, where n is an integer of at least two;
in accordance with the answers obtained in the obtaining, calculating scores for the respective domains, the scores being expressed as numerical values and related to the behavioral pattern of the patient;
in an n-dimensional space in which number lines used to show the scores for the respective domains correspond to coordinate axes, calculating a Euclidean distance between fixed-point coordinates and coordinates represented by the scores for the respective domains calculated in the calculating;
in accordance with the Euclidean distance calculated in the calculating, determining a dementia care burden level for the patient to be cared for; and
outputting the dementia care burden level determined in the determining.

12. A dementia care burden level determination program designed to cause a computer including a processor and memory to implement the dementia care burden level determination method according to claim 11.

13. A dementia treatment therapeutic effect determination device for determining a therapeutic effect of treatment for dementia in terms of a dementia care burden level for a patient to be cared for, the dementia treatment therapeutic effect determination device comprising:
an obtainment unit configured to obtain answers to respective questions about a behavioral pattern of the patient twice, once a first time round and once a second time round, the questions each belonging to one of n domains, where n is an integer of at least two;
a score calculator that, for each round the obtainment unit obtains the answers and in accordance with the answers obtained by the obtainment unit, calculates scores for the respective domains, the scores being expressed as numerical values and related to the behavioral pattern of the patient;
a distance calculator that calculates, for each round the score calculator calculates the scores, a Euclidean distance between fixed-point coordinates and coordinates represented by the scores for the respective domains in an n-dimensional space in which number lines used to show the scores for the respective domains correspond to coordinate axes;
a determination unit configured to determine a therapeutic effect of treatment for dementia in terms of the dementia care burden level for the patient to be cared for, in accordance with a difference between the Euclidean distance calculated the first time round by the distance calculator and the Euclidean distance calculated the second time round by the distance calculator; and
an output unit configured to output the therapeutic effect of the treatment for dementia determined by the determination unit.

14. The dementia treatment therapeutic effect determination device according to claim 13,
wherein the fixed-point coordinates correspond to coordinates obtained as a result of a combination, for which the dementia care burden level is preset to be highest, of answers to the respective questions and,
the determination unit determines the therapeutic effect of the treatment for dementia in terms of the dementia care burden level so that when two of the Euclidean distances, one shorter than the other and one longer than the other, are compared, the therapeutic effect of the treatment for dementia for the shorter Euclidean distance is not lower than the therapeutic effect of the treatment for dementia for the longer Euclidean distance.

15. The dementia treatment therapeutic effect determination device according to claim 13 or 14,
wherein the score calculator calculates the scores by calculating, for each of the domains, a sum of numerical values into which answers to questions belonging to the domain have been converted.

16. The dementia treatment therapeutic effect determination device according to claim 13 or 14,
wherein the score calculator calculates the scores by calculating, for each of the domains, a deviation value expressed as a Z-score or a T-score for the patient in a population distribution of sums calculated for respective patients, the sums each being a sum of numerical values into which answers to questions belonging to the domain have been converted.

17. The dementia treatment therapeutic effect determination device according to any one of claims 13 to 16,
wherein n is three.

18. The dementia treatment therapeutic effect determination device according to any one of claims 13 to 17, further comprising
an image generator that generates a three-dimensional image in which a position of the fixed-point coordinates, a position of the coordinates represented by the scores for the respective domains calculated the first time round by the score calculator, and a position of the coordinates represented by the scores for the respective domains calculated the second time round by the score calculator are visible together in the n-dimensional space,
wherein the output unit also displays the three-dimensional image generated by the image generator.

19. The dementia treatment therapeutic effect determination device according to any one of claims 13 to 18, further comprising:
question image storage that stores, for each of the questions, a question image containing the question and a plurality of answer choices to the question;
a reception unit configured to receive, for each of the question images stored in the question image storage, an answer choice selected from the plurality of answer choices contained in the question image; and
numerical value storage that stores, for each of the question images stored in the question image storage, numerical values and the plurality of answer choices contained in the question image, the numerical values each being associated with a different one of the plurality of answer choices,
wherein the output unit also displays each of the question images stored in the question image storage,
the obtainment unit obtains each answer by obtaining, as an answer, the answer choice received by the reception unit, and
the score calculator calculates the scores in accordance with the numerical values stored in the numerical value storage in association with the answer choices corresponding to the answers obtained by the obtainment unit.

20. The dementia treatment therapeutic effect determination device according to claim 19,
wherein each of the question images contains five answer choices, and
each of the question images also contains three illustrations for three respective answer choices among the five answer choices, the two remaining answer choices being associated with the highest numerical value and the lowest numerical value in the numerical value storage.

21. A dementia treatment therapeutic effect determination method of determining a therapeutic effect of treatment for dementia in terms of a dementia care burden level for a patient to be cared for, the dementia treatment therapeutic effect determination method comprising:
obtaining answers to respective questions about a behavioral pattern of the patient twice, once a first time round and once a second time round, the questions each belonging to one of n domains, where n is an integer of at least two;
for each round the answers are obtained in the obtaining and in accordance with the answers obtained in the obtaining, calculating scores for the respective domains, the scores being expressed as numerical values and related to the behavioral pattern of the patient;
for each round the scores are calculated in the calculating, calculating a Euclidean distance between fixed-point coordinates and coordinates represented by the scores for the respective domains calculated in the calculating, in an n-dimensional space in which number lines used to show the scores for the respective domains correspond to coordinate axes;
in accordance with a difference between the Euclidean distance calculated the first time round in the calculating and the Euclidean distance calculated the second time round in the calculating, determining a therapeutic effect of treatment for dementia in terms of the dementia care burden level for the patient to be cared for; and
outputting the therapeutic effect of the treatment for dementia determined in the determining.

22. A dementia treatment therapeutic effect determination program designed to cause a computer including a processor and memory to implement the dementia treatment therapeutic effect determination method according to claim 21.
